# EUROPEAN PATENT SPECIFICATION

(11) **EP 1 295 869 B1**
(45) Date of publication and mention of the grant of the patent: **27.07.2005**
(21) Application number: 02078056.5
(22) Date of filing: 12.02.1998
(51) Int. Cl.: C07C 253/30, C07C 255/46

(54) **Compounds and method for preparing substituted 4-phenyl-4-cyanocyclohexanoic acids**
Verbindungen und Verfahren zur Herstellung substituierter 4-Phenyl-4-Cyanocyclohexansäuren
Composés et procédé de préparation d'acides 4-phényl-4-cyanocyclohéxanoiques substitués

(30) Priority: 12.02.1997 US 37608 P
(43) Date of publication of application: 26.03.2003
(62) Divisional of application: 98908534.5
(73) Proprietor: SmithKline Beecham Corporation, Philadelphia, PA 19103 (US)
(72) Inventor: Allen, Andrew, Exton, PA 19341 (US); Diederich, Anne Marie, Downingtown, PA 19335 (US); Liu, Li, Collegeville, PA 19426 (US); Mendelson, Wilford, Kind of Prussia, PA 19406 (US)
(74) Representative: Rutter, Keith

(56) References cited:
- US-A- 5 552 438
- US-A- 5 602 157
- US-A- 5 602 173
- CHRISTENSEN S B ET AL: "1,4-Cyclohexanecarboxylates: Potent and Selective Inhibitors of Phosphodiesterase 4 for the Treatment of Asthma" JOURNAL OF MEDICINAL CHEMISTRY, AMERICAN CHEMICAL SOCIETY. WASHINGTON, US, vol. 41, 1998, pages 821-835, XP002173934 ISSN: 0022-2623

## Description

### Scope of the Invention

This invention covers intermediates and a synthetic route for making 4-cyano-4-(3-cyclopentyloxy-4-methoxyphenyl)cyclohexanoic acid and its analogs. This acid and its named analogs are selective for inhibiting the catalytic site in the phosphodiesterase isoenzyme denominated IV (PDE IV hereafter) and as such the acids are useful in treating a number of diseases which can be moderated by affecting the PDE IV enzyme and its subtypes.

### Area of the Invention

Bronchial asthma is a complex, multifactorial disease characterized by reversible narrowing of the airway and hyper-reactivity of the respiratory tract to external stimuli.

Identification of novel therapeutic agents for asthma is made difficult by the fact that multiple mediators are responsible for the development of the disease. Thus, it seems unlikely that eliminating the effects of a single mediator will have a substantial effect on all three components of chronic asthma. An alternative to the "mediator approach" is to regulate the activity of the cells responsible for the pathophysiology of the disease.

One such way is by elevating levels of CAMP (adenosine cyclic 3',5'-monophosphate). Cyclic AMP has been shown to be a second messenger mediating the biologic responses to a wide range of hormones, neurotransmitters and drugs; [Krebs Endocrinology Proceedings of the 4th International Congress Excerpta Medica, 17-29, 1973]. When the appropriate agonist binds to specific cell surface receptors, adenylate cyclase is activated, which converts Mg⁺²-ATP to cAMP at an accelerated rate.

Cyclic AMP modulates the activity of most, if not all, of the cells that contribute to the pathophysiology of extrinsic (allergic) asthma. As such, an elevation of cAMP would produce beneficial effects including: 1) airway smooth muscle relaxation, 2) inhibition of mast cell mediator release, 3) suppression of neutrophil degranulation, 4) inhibition of basophil degranulation, and 5) inhibition of monocyte and macrophage activation. Hence, compounds that activate adenylate cyclase or inhibit phosphodiesterase should be effective in suppressing the inappropriate activation of airway smooth muscle and a wide variety of inflammatory cells. The principal cellular mechanism for the inactivation of cAMP is hydrolysis of the 3'-phosphodiester bond by one or more of a family of isozymes referred to as cyclic nucleotide phosphodiesterases (PDEs).

It has now been shown that a distinct cyclic nucleotide phosphodiesterase (PDE) isozyme, PDE IV, is responsible for cAMP breakdown in airway smooth muscle and inflammatory cells. [Torphy, "Phosphodiesterase Isozymes: Potential Targets for Novel Anti-asthmatic Agents" in New Drugs for Asthma, Bames, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. Moreover, the beneficial effects of PDE IV inhibitors are markedly potentiated when adenylate cyclase activity of target cells is elevated by appropriate hormones or autocoids, as would be the case *in vivo.* Thus PDE IV inhibitors would be effective in the asthmatic lung, where levels of prostaglandin E₂ and prostacyclin (activators of adenylate cyclase) are elevated. Such compounds would offer a unique approach toward the pharmacotherapy of bronchial asthma and possess significant therapeutic advantages over agents currently on the market.

The process and intermediates of this invention provide a means for making certain 4-substituted-4-(3,4-disubstitutedphenyl)cyclohexanoic acids which are useful for treating asthma, and other diseases which can be moderated by affecting the PDE IV enzyme and its subtypes. The final products of particular interest are fully described in U.S. patent 5,552,483 issues 03 September 1996.

### Summary of the invention

This invention relates a method for making a compound of formula I 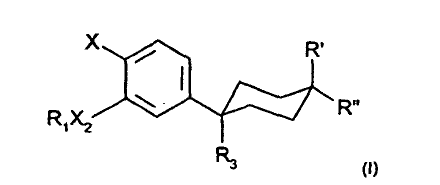 R₁ is -(CR₄R₅)ᵣR₆ wherein the alkyl moieties may be unsubstituted or substituted with one or more halogens;
r is 0 to 6;
R₄ and R₅ are independently selected from hydrogen or a C₁₋₂ alkyl;
R₆ is C₃₋₆ cycloalkyl wherein the cycloalkyl group may be unsubstituted or substituted by 1 to 3 methyl groups or one ethyl group;
X is YR₂;
X₂ is O;
Y is O or S(O)_{m'};
m' is 0, 1, or 2;
R₂ is independently selected from -CH₃ or -CH₂CH₃ unsubstituted or substituted by 1 or more halogens; R₃ is CN; and
R' and R" are independently hydrogen or -C(O)OH;
which process comprises
a) reacting a compound of formula II 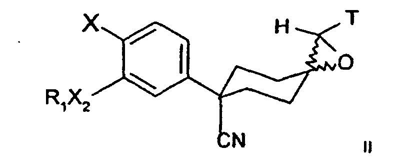 where R₁, R₃, X₂, and X are the same as in formula (I), and T is CN or SO₂R where R is C₁₋₆alkyl or _{C₀₋3}alkylphenyl, with at least about 1.5 equivalents of a Lewis acid at a temperature between about 60° and 100° C optionally under an inert atmosphere;
   wherein the compound of formula (II) is prepared by
b) reacting a ketone of formula A 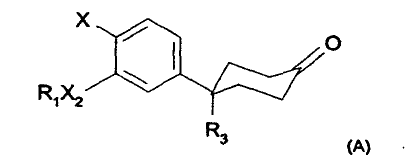 wherein R₁, R₃, X₂ and X are the same as in formula (I) with a molar excess of chloroacetonitrile, an inorganic base, and a catalytic amount of benzyltriethylammonium chloride in a water miscible solvent;
   wherein the compound of formula (A) is prepared by
c) decarboxylating a beta-keto ester of formula B 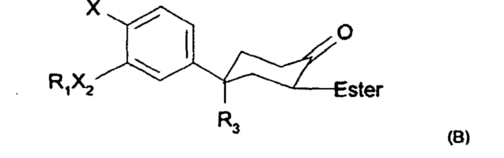 wherein R₁, R₃, X₂ and X are the same as in formula (I) by heating a solution of the beta-keto ester with dimethylsulfoxide to about 150°C; wherein the beta-keto ester of formula B is prepared by
d) cyclizing a pimelate of formula C 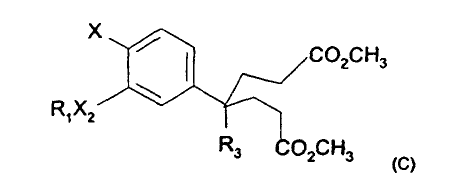 wherein R₁, R₃, X₂ and X are the same as in formula (I) with about 2 equivalents of a strong base at about
   75° C, wherein the compound of formula C is prepared by
e) reacting a nitrile of formula D 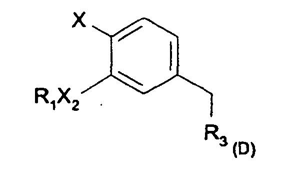 wherein R₁, R₃, X₂ and X are the same as in formula (I) with an alkyl acrylate in a 3-4-fold molar excess in the presence of a catalytic amount of Triton-B, wherein formula (D) compounds are prepared by
f) reacting a compound of formula (E) 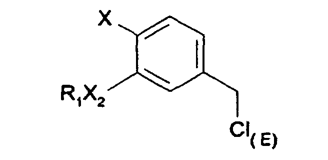 wherein R₁, X₂ and X are the same as in formula (I) with about a 50% molar excess of an alkali metal cyanide, wherein formula (E) compounds are prepared by:
g) reacting an alcohol of formula (F) 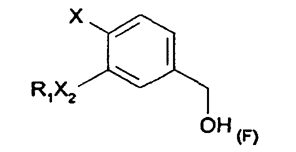 wherein R₁, X₂ and X are the same as in formula (I) with excess hydrochloric acid, wherein formula (F) compounds are prepared by:
h) reducing an aldehyde of formula (G) 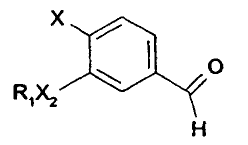 wherein R₁, X₂ and X are the same as in formula (I) wherein the reduction is carried out using an inorganic reducing agent.
In a further aspect, this invention relates to a process for preparing a compound of formula I(a) or I(b) or a mixture thereof 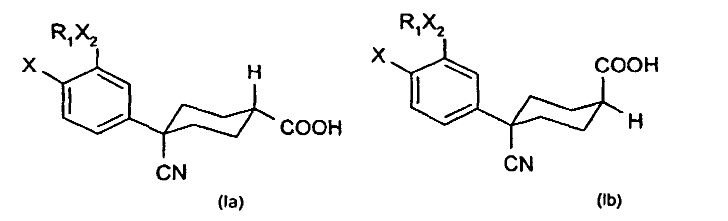 wherein
X is OR_{2,}
R₂ is independently selected from -CH₃ or -CH₂CH₃ optionally substituted by 1 or more halogens;
R₁ is -(CR₄R₅)ᵣR_{6,}
R₄ and R₅ are independently selected from hydrogen or a C₁₋₂ alkyl;
R₆ is C₃₋₆ cycloalkyl which may be optionally substituted by 1 to 3 methyl groups or one ethyl group; and X₂ is O;
which process comprises treating an acyl nitrile of formula (V) with water 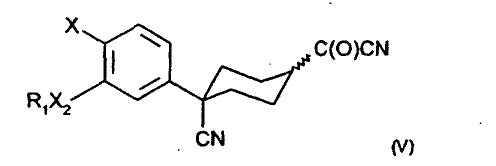 wherein X and R₁X₂ are the same as in formula (Ia) and (Ib).
In a still further aspect, this invention relates to a process for preparing a compound of formula (V) 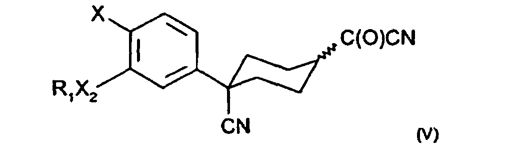 wherein
X is OR_{2,}
R₂ is independently selected from -CH₃ or -CH₂CH₃ optionally substituted by 1 or more halogens;
R₁ is -(CR₄R₅)ᵣR_{6,}
R₄ and R₅ are independently selected from hydrogen or a C₁₋₂ alkyl;
R₆ is C₃₋₆ cycloalkyl which may be optionally substituted by 1 to 3 methyl groups or one ethyl group; and X₂ is O;
which process comprises maintaining at about room temperature in solution for a time sufficient to effect the conversion, a compound of formula (Z) 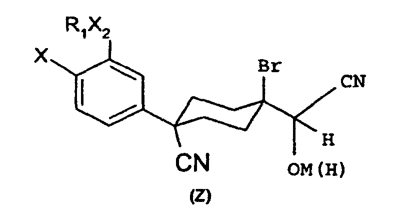 where X and R₁X₂ are the same as in formula (V) and M is U or Mg and the group (H) is hydrogen.
This invention also relates to a compound of formula (Z) per se 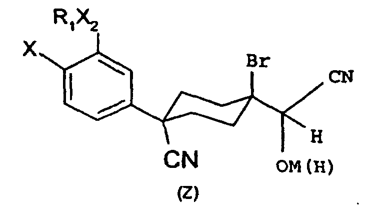 wherein
X is OR_{2,}
R₂ is independently selected from -CH₃ or -CH₂CH₃ optionally substituted by 1 or more halogens;
R₁ is -(CR₄R₅)ᵣR₆,
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O.
In a further aspect of the invention, a compound of formula (Z) may be made by treating an epoxide of formula (II) 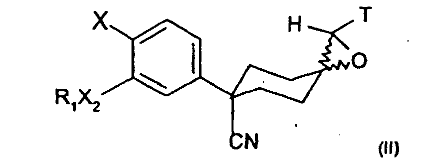 wherein X and R₁X₂ are the same as in formula (Z) and T is CN or SO₂R where R is C₁₋₆alkyl or C₀₋₃alkylphenyl with a Lewis acid under anhydrous conditions.
In a further aspect, there is provided a compound of formula (IV) 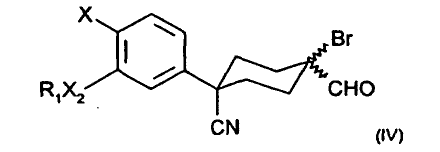 where
X is OR_{2,}
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and X₂ is O.
In a further aspect, this invention relates to a process for preparing a compound of formula (Ia) or (Ib) 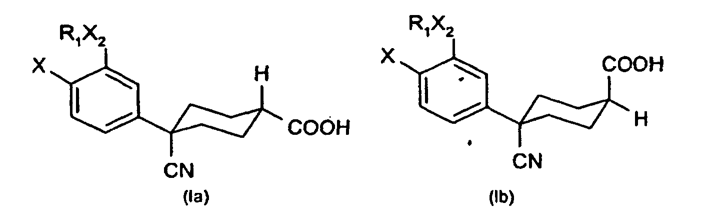 where
X is OR_{2,}
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and X₂ is O;
which process comprises treating an aldehyde of formula (IV) 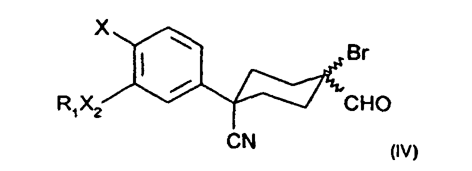 wherein X and R₁X₂ are the same as in formula I(a) and I(b) with an alkali metal cyanide.
In a further aspect, this invention relates to a process for preparing an acyl nitrile of formula (V) 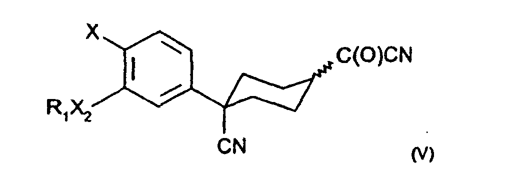 wherein
X is OR_{2,}
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises treating an aldehyde of formula (IV) 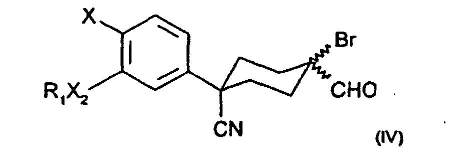 wherein X and R₁X₂ are the same as in formula (V) with LiCN and a catalytic amount of dimethyl formamide.
In a further aspect, this invention relates to a compound according to formula (VI) 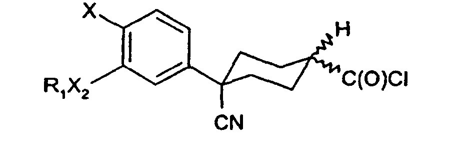 wherein
X is OR_{2,}
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O.

### Specific Embodiments of the Invention

This process involves a nine step synthesis for preparing certain 4-substituted-4-(3,4-disubstitutedphenyl) cyclohexanoic acids. The starting material is isovanillin, 3-hydroxy-4-methoxybenzaldehyde, or an analog thereof. "Analog" means another 3 and/or 4 position substituent conforming to the definitions of R₁, R₃, X₂ and X in the definition of formula (I).

The compounds which are made by this process are PDE IV inhibitors. They are useful for treating a number of diseases as described in U.S. patent 5,552,438 issued 3 September 1996.

The preferred compounds which can be made by this process are as follows:

Preferred R₁ substitutents for the compounds of all named formulas are CH₂-cyclopropyl, CH₂-C₅₋₆ cycloalkyl, C₄₋₆ cycloalkyl unsubstituted or substituted with OHC₇₋₁₁ polycycloalkyl, (3- or 4-cyclopentenyl), phenyl, tetrahydrofuran-3-yl, benzyl or C₁₋₂ alkyl unsubstituted or substituted by 1 or more fluorines, -(CH₂)₁₋₃C(O)O(CH₂)₀₋₂CH₃, -(CH₂)₁₋₃O(CH₂)₀₋₂CH₃, and -(CH₂)₂₋₄OH.

Preferred X groups for Formula (I), (II) or (A) are those wherein X is YR₂ and Y is oxygen. The preferred X₂ group for Formula (I) is that wherein X₂ is oxygen. Preferred R₂ groups are a C₁₋₂ alkyl unsubstituted or substituted by 1 or more halogens. The halogen atoms are preferably fluorine and chlorine, more preferably fluorine. More preferred R₂ groups are those wherein R₂ is methyl, or the fluoro-substituted alkyls, specifically a C₁₋₂ alkyl, such as a -CF₃, -CHF₂, or -CH₂CHF₂ moiety. Most preferred are the -CHF₂ and -CH₃ moieties.

Most preferred are those compounds wherein R₁ is -CH₂-cyclopropyl, cyclopentyl, 3-hydroxycyclopentyl, methyl or CF₂H; X is YR₂; Y is oxygen; X₂ is oxygen; and R₂ is CF₂H or methyl; and R₃ is CN.

A representative schematic of this process is set out in Scheme I. This graphical representation uses specific examples to illustrate the general methodology used in this invention.

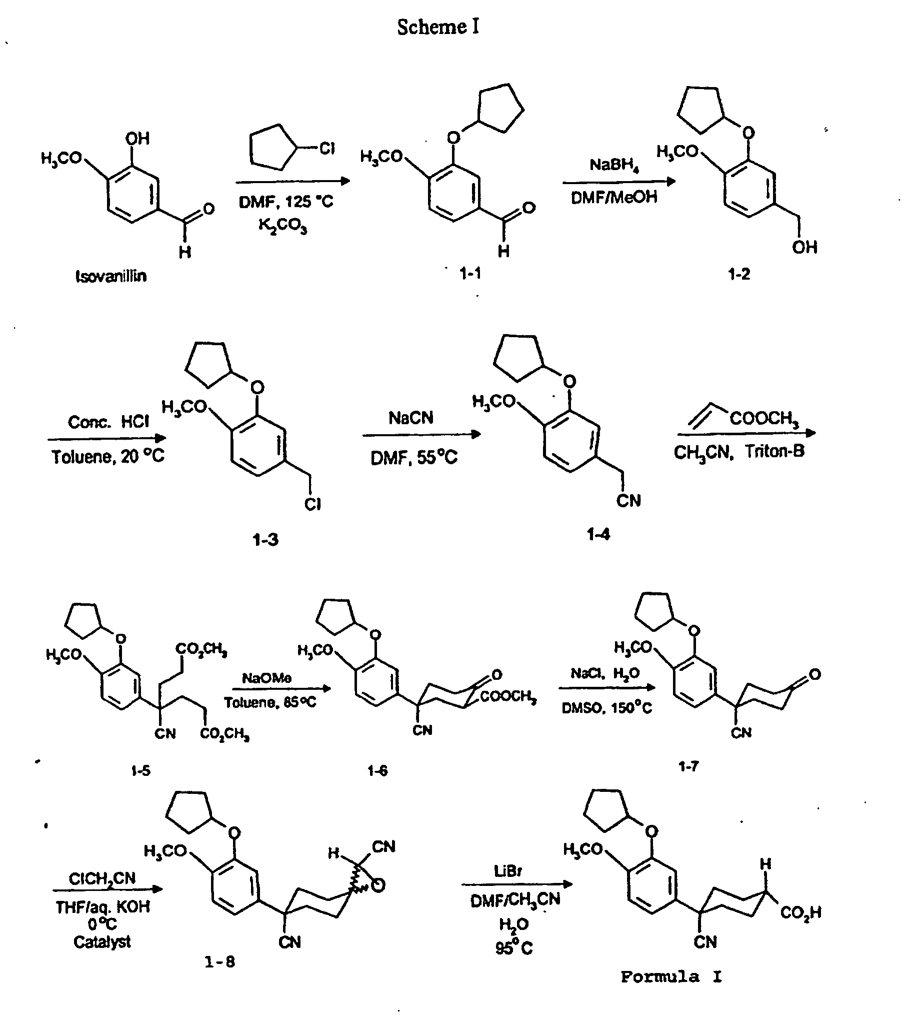

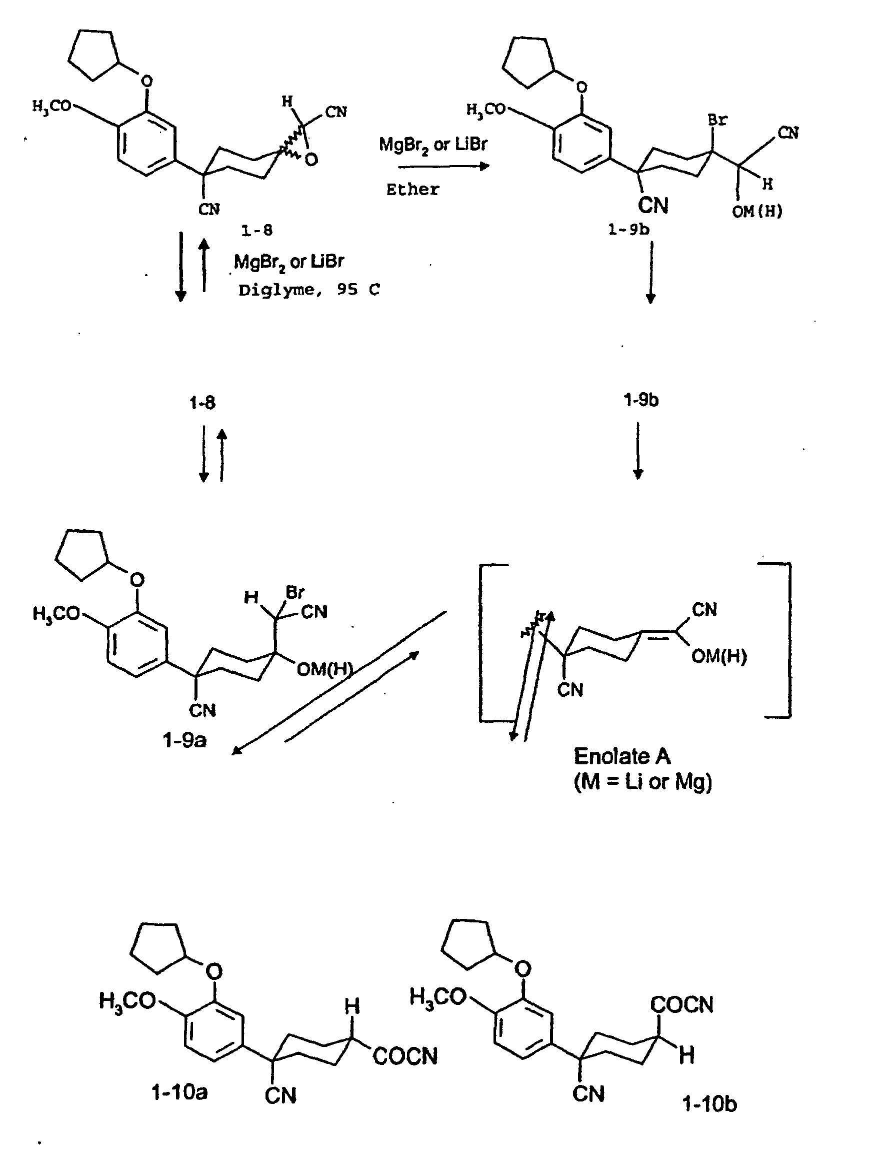

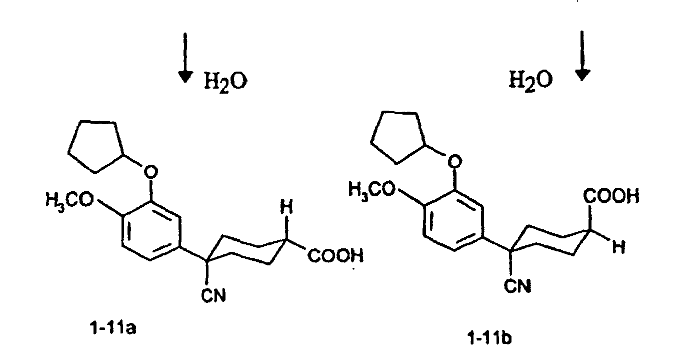

Referring to Scheme I, isovanillin, 3-hydroxy-4-methoxybenzaldehyde, is a readily available starting material. It can be alkylated with an R₁X moiety (X = Cl, Br, and I) as represented by cyclopentyl chloride. The reaction vessel is first flushed with an inert gas, for example nitrogen. A polar solvent such as DMF is then added to the vessel, then the isovanillin, then the R₁X adduct, and some base. About 2 equivalents of the R₁X adduct versus the isovanillin are used. Likewise about 2 equivalents of base are used, again relative to the isovanillin. The base can be any inorganic base or a carbonate. Here it is illustrated by potassium carbonate. The vessel contents are heated to about 125° C for about 90 to 120 minutes in which time the reaction will have gone to completion. The vessel contents are cooled to ambient temperature, filtered to remove the inorganic salts, and washed with an alcohol such as methanol. This filtrate contains the aldehyde, labeled 1-1.

The aldehyde is then reduced to the alcohol using an inorganic reducing agent. To do this the filtrate from the foregoing reaction is treated with sodium borohydride and after workup affords the desired alcohol, 1-2 in 97% overall yield from isovanillin. This is achieved by cooling the filtrate to about 0° C after which a reducing agent, here sodium borohydride, is added. About 0.25 to 0.5 equivalents of this reducing agent is used. The temperature is keep at about 0° C during the addition of the reducing agent and for about 30 to 40 minutes thereafter. Then the temperature is allowed to rise to about room temperature after which about one-half an equivalent of HCl is added to the reaction vessel. The alcohol is then extracted into an organic solvent, toluene is illustrated, and washed with dilute sodium bicarbonate.

The top organic layer containing the alcohol is then treated with excess concentrated hydrochloric acid at ambient temperature to afford, after workup, the desired benzyl chloride 1-3. The chloride is isolated as a w/w solution in an amide solvent, DMF is illustrated, and treated with about a 50% molar excess of sodium cyanide at a mildly elevated temperature, here illustrated as 55° C. This affords the desired nitrile 1-4. The nitrile is isolated as a w/w solution in an appropriate solvent such as anhydrous acetonitrile and used directly in the next step.

The nitrile solution is charged with methyl acrylate. It is cooled to about - 10° C, and slowly treated with a catalytic amount of Triton-B in the same solvent as used to dissolve the nitrile. The methyl acrylate is added in a 3 to 4-fold excess. The reaction is complete within 30 to 45 minutes after which the acrylate addition, the pimelate product, 1-5, is isolated as a w/w solution in toluene and treated with about 2 equivalents of sodium methoxide at about 75° C to give the β-keto-ester product, 1-6 . The reaction solution is cooled and neutralized to pH 7 with mineral acid such as 6N hydrochloric acid. The solution is charged with dimethyl sulfoxide, sodium chloride, water, and heated, for example to about 150° C, to effect the decarboxylation to give 1-7. The ketone, 1-7, is isolated from the solvent system as an off-white solid.

The dicarbonitrile 1-8 is prepared from the ketone by treating the ketone with chloroacetonitrile in the presence of an inorganic base and a catalytic amount of benzyltriethylammonium chloride (BTEAC). The ketone is charged into a mixture of strong base (aqueous potassium hydroxide) and a water miscible solvent such as tetrahydrofuran. A slight excess of chloroacetonitrile is added at reduced temperature, about 0° C or thereabouts. The reaction is maintained at about that temperature for the duration of the reaction, usually about 1 hour. The product is isolated and usually it is crystalline.

The dicarbonitrile is converted to the cyclohexanecarboxylic acid using a Lewis acid catalyst; water is also needed to drive the reaction to the acid. Without water intermediates 1-10a and 1-10b may dimerize. This reaction is carried out by charging a vessel with solvents, in this instance exemplified by DMF, acetonitrile and water, and the Lewis acid (about 1.5 equivalents), LiBr is illustrated, sweeping the vessel with an inert gas, adding the dicarbonitrile IIa or IIb, or a mixture of IIa and IIb and heating the vessel and its contents to about 100° C for a number of hours, 8 hours being an example. The acid is isolated by conventional means.

It should be noted that this reaction, that is the conversion of the epoxide to the acid, involves several intermediates which do not need to be isolated. It has been found that treating the epoxide with LiBr yields intermediates 1-9a and 1-9b. Intermediate 1-9a is formed when LiBr is added to the reaction pot. But intermediate 1-9a converts back to the epoxide under the recited reaction conditions. Intermediate 1-9b is also formed but apparently reacts rapidly to form intermediates such as enolate A, 1-10a and 1-10b etc leading to product. So it appears 1-9a and 1-9b are formed, but that 1-9a converts back to the epoxide which ultimately forms 1-9b which is then converted to other intermediates enroute to forming the acids of 1-11a and 1-11b. Parenthetically the designation "OM(H)" in 1-9a and 1-9b means the metal salt of the alcohol or the alcohol per se, depending on the reaction conditions. Intermediate 1-9b is believed to convert to the acyl nitriles of formulas 1-10a and 1-10b via the proposed bracketed intermediate. The existence of the proposed bracketed intermediate (enolate) has not been fully confirmed. And while the acyl nitriles of 1-10a and 1-10b have not been directly observed, indirect evidence exists for these compounds by virtue of the fact the bis-condensation product dimer B was isolated and is analogous to reported compounds where a similar bis-condensate is the product of an acyl nitrile.

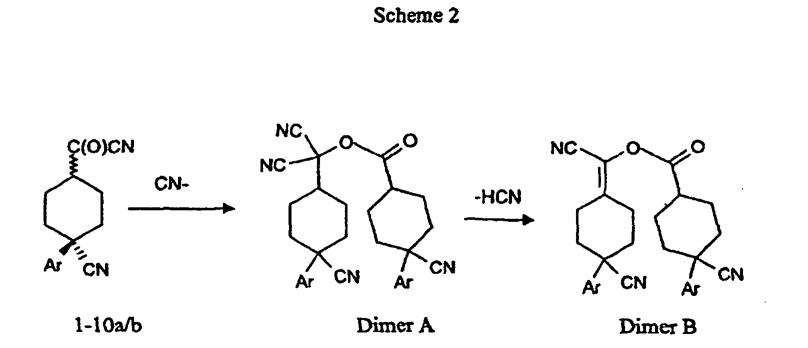

Dimers such as dimer A are known to form from the likes of acyl nitriles-1-10a/b in the presence of HCN (Thesing, J.; Witzel, D.; Brehm, A. *Angew Chem..* 1956, 68, 425; and Hunig, S.; Schaller, R. *Angew. Chem. Int. Ed. Engl..* 1982, 21,36).

And in addition, authentic samples of intermediates 1-10a and 1-10b were prepared and found to convert to acids 1-11a and 1-11b when exposed to water. The equitorial isomer 1-10a converted to the acid in an equitorial/axial ratio of about 98:2 while the axial isomer 1-10a isomerized to a perponderance of the equitorial isomer 1-11a (77:23). It is believed the axial acyl nitrile converts to the equitorial acyl nitrile via the proposed bracketed enolate intermediate.

The second, following reaction scheme illustrates preparing the acids of formula (I) from the bromoaldehyde of formula (IV).

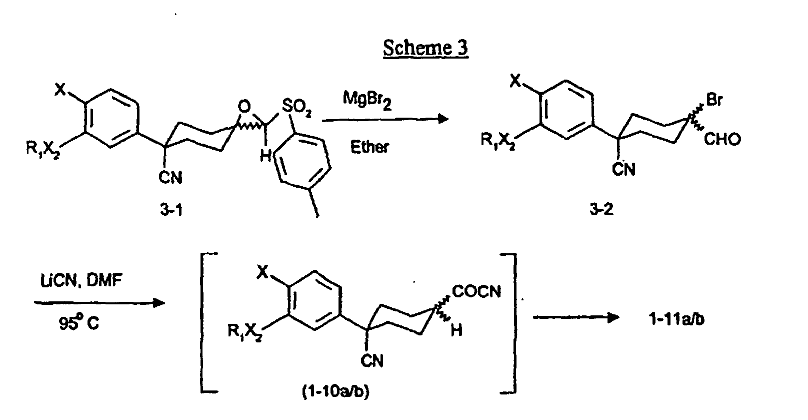

The following examples are provided to illustrate specifics of the invention, not to limit it. What is reserved to the inventors is set forth in the claims appended hereto.

### Specific Examples

### Reference Example 1

### Preparation of 3-cyclopentyloxy-4-methoxybenzaldehyde

A 12 liter round bottom flask equipped with an overhead stirrer, internal thermometer, and a reflux condensor equipped with a nitrogen inlet was flushed with nitrogen. The flask was charged with dimethylformamide (2.4 L), isovanillin (350 g, 2.3 mol, 1 equivalent), cyclopentyl chloride (481 g, 4.6 mol, 2.0 equivalent) and potassium carbonate (634 g, 4.6 mol, 2.0 equivalents). The vigorously stirred suspension was heated to 125 °C for two hours or until the disappearance of isovanillin. The reaction was cooled to 20-30 °C and filtered to remove the inorganic salts. The filter cake was rinsed with methanol (1.0 L).

The clear, light-brown filtrate (DMF and methanol) containing the product, 3-cyclopentyloxy-4-methoxybenzaldehyde, was used directly in the next step (100 % solution yield).

### Reference Example 2

### Preparation of 3-cyclopentyloxy-4-methoxybenzyl alcohol.

A 12 liter round bottom flask equipped with an overhead stirrer, internal thermometer, and a reflux condensor equipped with a nitrogen inlet was flushed with nitrogen. The flask was charged with dimethylformamide (2.4 L), methanol (1.0 L), and 3-cyclopentyloxy-4-methoxybenzaldehyde (506 g, 2.3 mol, 1 equivalent). The contents of the flask were cooled to 0 to 5 °C followed by the addition of sodium borohydride (32.2 g, 0.85 mol, 0.37 equivalents). The reaction was maintained at 0 to 5 °C for 30 minutes, and warmed to 20 to 25 °C for an additional 2 hours or until the disappearance of the aldehyde. A solution of 6N hydrochloric acid (195 mL, 1.17 mol, 0.51 equivalents) was added over 20 minutes. The reaction was concentrated under reduced pressure, and cooled to 20 to 25 °C.

The flask was charged with deionized water (1.9 L) and toluene (1.9 L). The layers were separated, the organic layer was isolated, and washed twice with deionized water (2 x 800 mL). The product, 3-cyclopentyloxy-4-methoxybenzyl alcohol was collected as a solution in toluene (97% solution yield) and used directly in the next step.

### Reference Example 3

### Preparation of 4-Chloromethyl-2-cyclopentyloxy-1-methoxybenzene.

A 12 liter round bottom flask equipped with an overhead stirrer, internal thermometer, and a reflux condensor equipped with a nitrogen inlet was flushed with nitrogen. The flask was charged with 3-cyclopentyloxy-4-methoxybenzyl alcohol (495 g, 2.2 mol, 1 equivalent) in a solution of toluene. To the vigorously stirred reaction at 22 °C was added concentrated hydrochloric acid (600 g, 2.75 equivalents). The reaction was maintained at 20 to 25 °C for 30 minutes. The top organic layer was isolated and the bottom acidic layer was discarded. To the top organic layer was charged a solution of 10% sodium bicarbonate (550 g, 0.65 mol, 0.36 equivalents) and t-butyl methyl ether (814 g). The contents of the flask were vigorously stirred, and allowed to settle. The product, 4-chloromethyl-2-cyclopentyloxy-1-methoxybenzene, was isolated as a solution in toluene and *t*-butyl methyl ether (96.8% solution yield). This was used directly in the next step.

### Reference Example 4

### Preparation of 4-Cyanomethyl-2-cyclopentyloxy-1-methoxybenzene.

A 12 liter round bottom flask equipped with an overhead stirrer, and a distillation apparatus was flushed with nitrogen. The flask was charged with 4-chloromethyl-2-cyclopentyloxy-1-methoxybenzene (519 g, 2.15 mol, 1.0 equivalents) in a solution of toluene and t-butyl methyl ether. The reaction was concentrated under reduce pressure to a residue. To the 12 liter flask was charged DMF (1.44 kg) and sodium cyanide (142 g, 2.9 mol, 1.35 equivalents). The reaction was heated to 55 °C for 6 hours or until deemed complete by the disappearance of the benzyl chloride. The reaction was concentrated under reduced pressure to a residue. To the flask was charged t-butyl methyl ether (2.30 kg) and deionized water (800 mL). The contents of the flask were vigorously stirred, and allowed to settle. The top organic layer was isolated, washed three times with deionized water (3 x 800 mL), and concentrated under atmospheric pressure to a residue. To the flask was added acetonitrile (1.26 kg) and the distillation was continued until an additional 400 mL of solvent was collected. The product, 4-cyanomethyl-2-cyclopentyloxy-1-methoxybenzene, was isolated as a solution in acetonitrile (92.2% yield). This was used directly in the next step.

### Reference Example 5

### Preparation of Dlmethyl-4-cyano-4-(3-cyclopentyloxy-4-methoxyphenyl)pimelate

A 12 liter round bottom flask equipped with an overhead stirrer, internal thermometer, and a reflux condensor equipped with a nitrogen inlet was flushed with nitrogen. The flask was charged with a solution of 4-cyanomethyl-2-cyclopentyloxy-1-methoxybenzene (460 g, 1.99 mol, 1.0 equivalent) in acetonitrile, and methyl acrylate (520 g, 6.0 mol, 3.0 equivalents). The contents of the flask was cooled to -10 °C. A pressure equalizing addition funnel was charged with acetonitrile (1.1 L) and benzyltrimethyl ammonium hydroxide (a 40% w/w solution in methanol, 25 g, 0.06 mol, 0.03 equivalents). The contents of the addition funnel was added to the flask. An exotherm was observed, and after stirring for 30 minutes the contents of the flask were cooled to 20 °C. The reaction was concentrated under reduced pressure to a residue. To the residue was added toluene (2.6 L). This solution of dimethyl-4-cyano-4-(3-cyclopentyloxy-4-methoxy-phenyl)pimelate (90% solution yield) was used directly in the next step.

### Reference Example 6

### Preparation of 4-Cyano-4-(3-cyclopentyloxy-4-methoxyphenyl)cyclohexan-1-one

A 12 liter round bottom flask equipped with an overhead stirrer, internal thermometer, and a reflux condensor equipped with a nitrogen inlet was flushed with nitrogen. The flask was charged with a solution of dimethyl-4-cyano-4-(3-cyclopentyloxy-4-methoxy-phenyl)pimelate (720 g, 1.78 mol, 1 equivalent) in toluene, and sodium methoxide (25 wt% in methanol, 545 g, 2.67 mol, 1.5 equivalents). The reaction was heated to 70 to 75 °C for 2 hours or until deemed complete by the disappearance of the pimelate. The reaction was cooled to 25 °C. A solution of 6N hydrochloric acid was added in order to adjust the pH to 6.8 - 7.2. The reaction was concentrated under vacuum to a residue. The flask was charged with dimethylsulfoxide (3.3 L), deionized water (250 mL) and sodium chloride (250 g).

The contents of the flask were heated to 145-155 °C, and held at this temperature for 2 hours. The reaction was cooled and concentrated under vacuum to a residue. To the residue was added water (1.9 L), ethyl acetate (1.25 L), and t-butyl methyl ether (620 mL). The solution was stirred and allowed to settle. The layers were separated, and the aqueous layer was re-extracted with ethyl acetate (1.25 L). The combined organic layers were washed twice with deionized water (2 X 2.5 L). The organic layer was isolated and concentrated under reduced pressure to a residue. To this residue was added isopropanol (1.66 L) and heated to produce a solution followed by the slow addition of hexanes (1.66 L). The suspension was cooled to 5 °C over 30 minutes, and held at 0 to 5 °C for two hours. The product was filtered and washed with 50-50 isopropanol-hexanes (840 mL) mixture at 0 °C. The product was dried to afford 4-cyano-4-(3-cyclopentyloxy-4-methoxyphenyl)cyclohexan-1-one (315 g, 56 % from the pimelate).

### Example 7

### Preparation of cis-(+/-)-6-[3-(cyclopentyloxy)-4-methoxyphenyl]-1-oxobicyclo[2.5]octane-2,6-dicarbonitrile.

A 5 liter round bottom flask equipped with an overhead stirrer, internal thermometer, and a nitrogen inlet was flushed with nitrogen. The flask was charged with 50% potassium hydroxide (220 g) and tetrahydrofuran (550 mL). While stirring at room temperature, benzyltriethylammonium chloride (8.1 g, 0.035 mol, 0.05 equivalent) was added. The solution was cooled to 0 °C. To a pressure equalizing addition funnel was charged a solution containing tetrahydrofuran (550 mL), 4-cyano-4-(3-cyclopentyloxy-4-methoxyphenyl)cyclohexan-1-one (230 g, 0.73 mol, 1.0 equivalent), and chloroacetonitrile (59 g, 0.78 mol, 1.07 equivalent) at room temperature. While stirring the flasks contents at 0 °C, the solution in the pressure addition funnel was added over 15 minutes. The temperature was maintained between 0 and 5 °C, and stirred for one hour. The reaction was warmed to 25 °C, diluted with water (900 mL), and ethyl acetate (900 mL). The solution was stirred and allowed to settle for 30 minutes. The layers were separated, the organic layer was isolated, and concentrated by vacuum distillation to a residue. Methanol (540 mL) was added and the solution was heated to 40 °C. While cooling to 20 ° C over 90 minutes, hexanes (540 mL) was added. Cooling was continued, and the product began to crystallize at 10 °C. The suspension was then cooled to -5 °C and held at -5-0 °C for two hours. The product was filtered and washed with a 50-50 methanol-hexanes mixture (300 mL) at 0 °C. The product was dried to afford cis-(+/-)-6-[3-(cyclopentyloxy)-4-methoxyphenyl]-1-oxobicyclo[2.5]octane-2,6-dicarbonitrile (190 g, 73 %) as a white crystalline solid.

### Example 8

### Preparation of 1-9a

In a stoppered 12 dram screw-top vial was added diglyme (5.92 g) and the epoxy nitrile (0.70 g, 1eq) of Example 7. This mixture was stirred with heating in an oil bath for 5 min. Then MgBr₂•6H₂O (0.906 g, 1.55 eq) was added. After 3 hr no starting material was detected. The reaction mixture was cooled, then mixed with 5% aqueous citric acid/ethyl acetate and the layers shaken and separated. The second extraction with ether/ethyl acetate gave some color extracted into organic layer; but the next extraction had no color. The organic fractions were combined and washed with water and brine and dried with MgSO₄. The product was crystallized from hexane; mp 151-152 °C.

Elemental analysis: C - 58.20, H - 5,82, Br - 18.44, N - 6.46; found C - 58.32, H - 5.73, Br - 18.48, N - 6.34. The structure was confirmed by X-ray structure determination of a crystalline sample obtained from methyl alcohol.

### Example 9

### Preparation of 1-9b

To Mg (0.189 g, 2.02 eq) (polished with mortar and pestle) in ether was added 1,2-dibromoethane (1.55 g, 2.06 eq) in a small volume of ether to initiate the Grignard. When most of the magnesium was consumed and no more evolution of ethane was observed, the reaction was stirred for an additional 0.5 hr at room temperature after which was added the epoxide of Example 7 (1.41 g; 1 eq) in a minimal amount of dry tetrahydrofuran at ambient temperature. After about 70 hr at room temperature there was obtained both the bromo cyanoalcohol (1-9b) and bromo cyanohydrin (1-9a) in a ratio of 6:1. The 1-9b product was isolated as an oil by prepartive HPLC. The structure was confirmed by carbon and proton NMR.

### Example 10

### Preparation of Compound 3-1 - Epoxysulfone

To a 25ml round-bottom flask equipped with a magnetic stir bar and a rubber septum was charged 1.00 g of 4-cyano-4-(3-cyclopentyloxy-4-methoxyphenyl)cyclohexan-1-one, 0.70 g of chloromethyl *p*-tolylsulfome, and 7 ml of tetrahydrofuran. This was stirred, then 3 ml of 50% w/w aqueous NaOH and benzyltrimeth ch (0.05g) was added. This suspension was vigorously stirred for 2 hours at room temperature. The reaction solution was transferred to a separatory funnel to which was added 50 ml of ethyl acetate and acidified with 6N HCl. The organic layer was retained, washed 2X with deionized water, dried with MgSO₄ and filtered to remove the salts.

### Example 11

### Preparation of Compound 3-2 - Bromoaldehyde

To magnesium (0.048g, 1.03 eq, 0.021 mol; polished with mortar and pestle) in ether was added 1,2-dibromoethane (0.40 g, 1.06 eq, 0.02 mol) under nitrogen. Two drops of iodine were added in ether to start the reaction, after which the reaction was heated gently. Once the Grignard had formed, the reaction flask was cooled to about 5 °C and the epoxysulfone of Example 10 (0.93 g, 1 eq, 0.002 mol) was added in ether/methylene chloride. The reaction was followed via TLC (conditions: silica gel with cyclohexane:toluene:acetonitrile:acetlc acid 40:40:20:4). The reaction was stirred at 5° for 2 hours. The product was isolated by adding water and ether/TBME to the reaction mixture, and seperating the organic layers. These were washed with water and brine and dried over MgSO₄. Evaporation gave an oil which was flash chromatographed over 40 g of silica gel using a mixture of hexane and ethyl acetate (5-40% ethyl acetate). This gave a clear oil (0.49 g) containing about equal proportions of the equitorial and axial isomers as determined by proton NMR. Mass specrum showed a molecular ion at m/e 405 containing 1 bromine atom [C₂₀H₂₄BrNO₃].

### Example 12

### Preparation of c-4-cyano-4-(3-cyclopentyloxy-4-methoxyphenyl)-r-cyclohexanecarboxylic acid.

A 5 liter round bottom flask equipped with an overhead stirrer, internal thermometer, and a reflux condensor equipped with a nitrogen inlet was flushed with nitrogen. The flask was charged with dimethylformamide (580 g), acetonitrile (480 g), lithium bromide (72 g, 0.83 mol, 1.62 equivalents) and deionized water (20 g, 1.1 mol, 2.2 equivalents). The solution was stirred under nitrogen at 25 - 30 °C followed by the addition of cis-(+/-)-6-[3-(pentyloxy)-4-methoxyphenyl]-1-oxobicyclo[2.5]octane-2,6-dicarbonitrile (180 g, 0.51 mol, 1.0 equivalent). The reactor cyclo was heated to 90 - 95 °C for 8 hours or until deemed complete by the disappearance of epoxy nitrile. The contents of the flask was cooled to 20 °C, followed by the addition of a sodium hydroxide solution (92 g of sodium hydroxide, 2.3 mol, 4.5 equivalents, dissolved in 200 mL of deionized water). The suspension was stirred at 20 °C for 30 minutes followed by the addition of sodium hypochlorite (600 mL, 0.46 mol, 0.9 equivalents). The contents of the flask were stirred for 90 minutes followed by the addition of t-butyl methyl ether (2.27 kg) and 6N HCl (644 mL, 3.86 mol, 7.5 equivalents). The bottom aqueous layer was back-extracted with t-butyl methyl ether (454 g), and the combined organic layer was washed four times with deionized water (4 X 800 mL). The organic layer was concentrated to a residue. To the flask was charged ethyl acetate (900 g) and heated to reflux. The contents of the flask was cooled to 50 °C followed by the addition of hexanes (672 g). The contents of the flask were cooled to 0 °C and held for 1 hour. The product was filtered and washed with cold ethyl acetate / hexanes (1/9, 175 g). The product was dried to afford *c*-4-cyano-4-(3-cyclopentyloxy-4-methoxyphenyl)-*r*-cyclohexanecarboxylic acid (125 g, 69 %) as an off-white powder.

### Example 13

### Preparation of c-4-cyano-4-(3-cyclopentyloxy-4-methoxyphenyl)-r-cyclohexanecarboxylic acid chloride

In a 1 neck flask equipped for nitrogen flow was combined *c*-4-cyano-4-(3-cyclopentyloxy-4-methoxyphenyl)-*r*-cyclohexanecarboxylic acid (1.372g, 1 eq, 0.004 mol) and oxalyl chloride (4.568g, 9eq, 0.036 mol). One drop of dimethyl formamide was then added. This mixture was stirred at ambient temperature overnight. After evaporation under high vacuum this yielded the captioned product.

### Example 14

### Preparation of Form 1-10a - 4-cyano-4-(3-cyclopentyloxy-4-methoxyphenyl)-r-cyclohexane acyl nitrile

In a flask a sample of the acid chloride (0.217 g, 0.006 mol, 1 eq) prepared in Example 12 was dissolved in CDCl₃ (2.34 mL). To this was solution (cooled to 5°C) was added trimethylsilyl cyanide (0.07g, 1.3 eq, 0.008 mol.) and a catalytic amount of Znl₂ (0.004g). This solution was refluxed overnight. This yielded 0.211g of the captioned product. IR: COCN, ν 2220 cm⁻¹; C=O, ν 1720cm⁻¹. The isomeric purity of 1-10a was determined by hydrolyzing the acyl nitrile in warm water, the product being essentially pure compound 1-11a.

### Example 15

### Preparation of Form 1-10b of 4-cyano-4-(3-cyclopentyloxy-4-methoxyphenyl)-r-cyclohexane acyl nitrile

In an experiment analogous to Example 14, the axial carboxylic acid was converted to the acid chloride using oxalyl chloride and catalytic amount of dimethyl formamide. This acid chloride was converted directly to the corresponding acyl nitrile, 1-10b, the isomer of the compound prepared in Example 14. The isomeric purity was assayed by hydrolyzing the acyl nitrile by stirring it in warm water for 20 hours. Analytical HPLC determination showed that > 96% of the product had the form of 1-10b.

## Claims

1. A process for preparing a compound of formula (I) 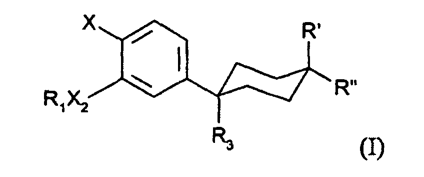 R₁ is -(CR₄R₅)ᵣR₆ wherein the alkyl moieties may be unsubstituted or substituted with one or more halogens;
r is 0 to 6;
R₄ and R₅ are independently selected from hydrogen or a C₁₋₂ alkyl;
R₆ is C₃₋₆ cycloalkyl wherein the cycloalkyl group may be unsubstituted or substituted by 1 to 3 methyl groups or one ethyl group;
X is YR₂;
X₂ is O;
Y is O or S(O)_{m'};
m' is 0, 1, or 2;
R₂ is independently selected from -CH₃ or -CH₂CH₃ unsubstituted or substituted by 1 or more halogens;
R₃ is CN; and
R' and R" are independently hydrogen or -C(O)OH;
which process comprises
a) reacting a compound of formula II 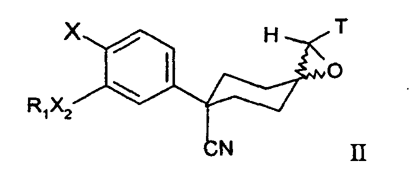 where R₁, R₃, X₂ and X are the same as in formula (I), and T is CN or SO₂R where R is C₁₋₆alkyl or C₀₋₃alkylphenyl, with at least about 1.5 equivalents of a Lewis acid at a temperature between about 60° and 100° C optionally under an inert atmosphere;
wherein the compound of formula (II) is prepared by
b) reacting a ketone of formula A 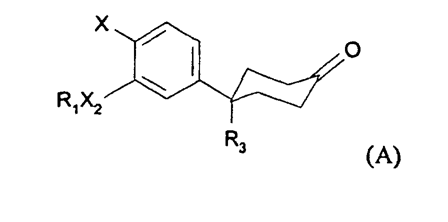 wherein R₁, R₃, X₂ and X are the same as in formula (I) with a molar excess of chloroacetonitrile, an inorganic base, and a catalytic amount of benzyltriethylammonium chloride in a water miscible solvent;
wherein the compound of formula (A) is prepared by
c) decarboxylating a beta-keto ester of formula B 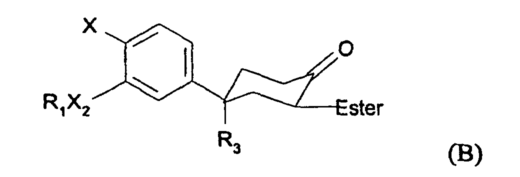 wherein R₁, R₃, X₂ and X are the same as in formula (I) by heating a solution of the the beta-keto ester with dimethylsulfoxide to about 150° C; wherein the beta-keto ester of formula B is prepared by
d) cyclizing a pimelate of formula C 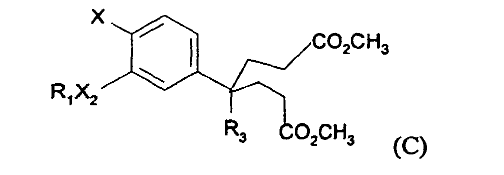 wherein R₁, R₃, X₂ and X are the same as in formula (I) with about 2 equivalents of a strong base at about 75° C, wherein the compound of formula C is prepared by
e) reacting a nitrile of formula D 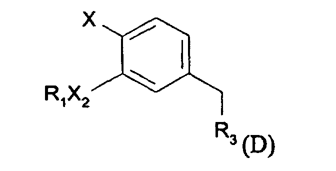 wherein R₁, R₃, X₂ and X are the same as in formula (I) with an alkyl acrylate in a 3-4-fold molar excess in the presence of a catalytic amount of Triton-B, wherein formula (D) compounds are prepared by
f) reacting a compound of formula (E) 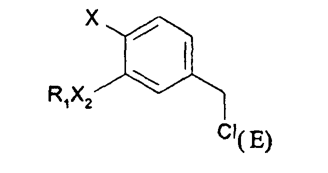 wherein R₁, X₂ and X are the same as in formula (I) with about a 50% molar excess of an alkali metal cyanide, wherein formula (E) compounds are prepared by;
g) reacting an alcohol of formula (F) 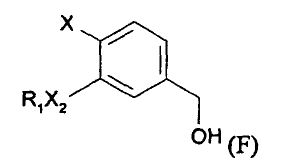 wherein R₁, X₂ and X are the same as in formula (I) with excess hydrochloric acid, wherein formula (F) compounds are prepared by;
h) reducing an aldehyde of formula (G) 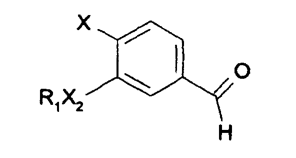
wherein R₁, X₂ and X are the same as in formula (I) wherein the reduction is carried out using an inorganic reducing agent.

2. The process of claim 1 wherein, in step a), the Lewis acid is lithium bromide, at least about 2 equivalents of water are present, and the reaction is heated to about 90-95 °C for up to about 8 hours.

3. The process of claim 1 wherein, in step b) the inorganic base is aqueous potassium hydroxide and the water-miscible solvent is tetrahydrofuran and the reaction is carried out at a temperature of about 0 °C.

4. The process of claim 1 wherein in step c) the strong base is sodium methoxide which is present in about 1.5 molar equivalents and the reaction is carried out at about 70 to 75 °C for up to 2 hours.

5. The process of claim 1 wherein steps c) and d) are combined by cyclizing the pimelate by reacting it with sodium methoxide decarboxylating the resulting beta-keto ester without isolating it by adding dimethylsulfoxide to the ester-containing solution and heating that solution to about 145 °C for up to 2 hours.

6. The process of claim 5 wherein after the cyclization reaction step is completed, the reaction mixture is cooled to about room temperature and the pH is adjusted to 6.8-7.2 before adding dimethylsulfoxide.

7. The process of claim 1, step e), wherein the alkyl acrylate is methyl acrylate.

8. The process of claim 1, step f), wherein the alkali metal cyanide is sodium cyanide and the reaction is carried out at about 55 °C.

9. The process of claim 1, step h), wherein the reducing agent is sodium borohydride.

10. The process of any one of claims 1-9 wherein, in formula I, R₁ is cyclopentyl; Y is oxygen; and R₂ is methyl.

11. A process for preparing a compound of formula I(a) or I(b) or a mixture thereof 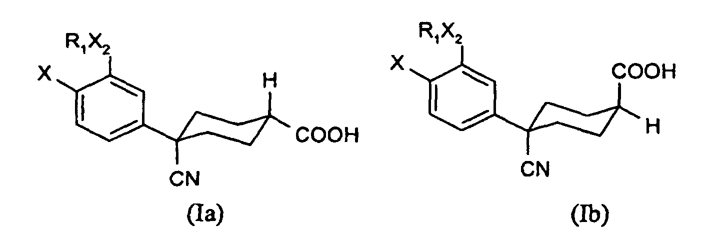 wherein
X is OR_{2,}
R₂ is independently selected from -CH₃ or -CH₂CH₃ optionally substituted by 1 or more halogens;
R₁ is -(CR₄R₅)ᵣR₆,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises treating an acyl nitrile of formula (V) with water 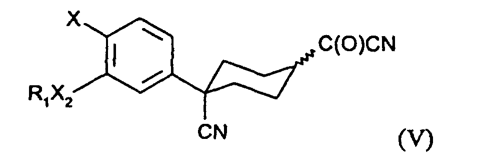 wherein X and R₁X₂ are the same as in formula (Ia) and (Ib).

12. The process of claim 11 wherein the compound of formula (V) is that of formula (Va) 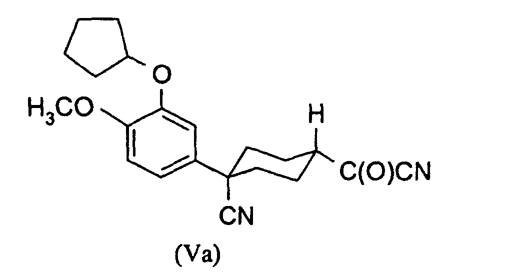 and results in the formation of a compound of formula (Ia).

13. The process of claim 11 wherein the compound of formula (V) is that of formula (Vb) 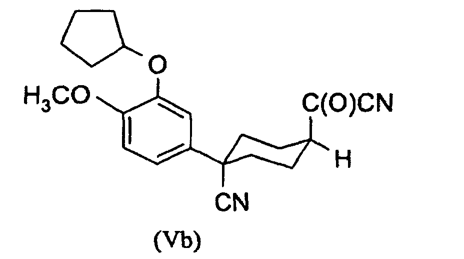 and results in the formation of a compound of formula (Ib).

14. A process for preparing a compound of formula (V) 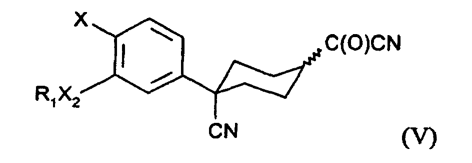 wherein
X is OR_{2,}
R₂ is independently selected from -CH₃ or -CH₂CH₃ optionally substituted by 1 or more halogens;
R₁ is -(CR₄R₅)ᵣR₆,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises maintaining at about room temperature in solution for a time sufficient to effect the conversion, a compound of formula (Z) 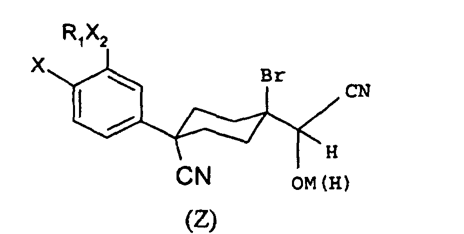 where X and R₁X₂ are the same as in formula (V) and M is Li or Mg and the group (H) is hydrogen.

15. The process of claim 14 wherein formula (Z) is that of figure 1 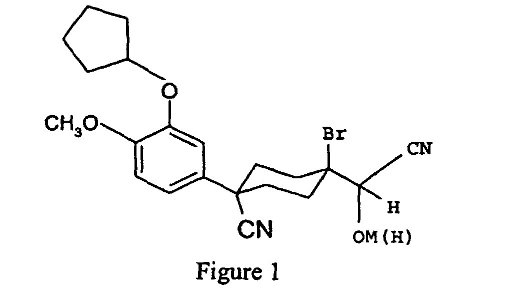 where M is Li or Mg and (H) is hydrogen.

16. A compound of formula (Z) 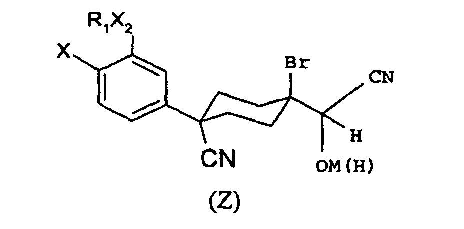 wherein
X is OR_{2,}
R₂ is independently selected from -CH₃ or -CH₂CH₃ optionally substituted by 1 or more halogens;
R₁ is -(CR₄R₅)rR₆,
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O.

17. A compound of formula (Z) according to claim 16 which is 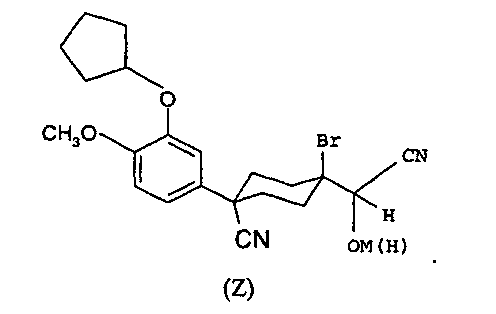 wherein M is Li or Mg and (H) is hydrogen.

18. A process for preparing a compound of formula (Z) 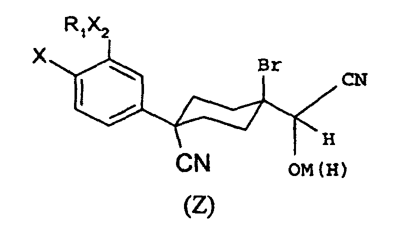 wherein
X is OR_{2,}
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises treating an epoxide of formula (II) 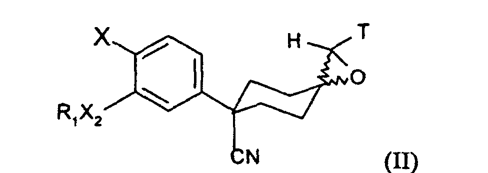 wherein X and R₁X₂ are the same as in formula (Z) and T is CN or SO₂R where R is C₁₋₆alkyl or C₀₋₃alkylphenyl with a Lewis acid under anhydrous conditions.

19. The process of claim 18 wherein the Lewis acid is LiBr.

20. The process of claim 18 or 19 wherein the epoxide of formula (II) has the structure represented by figure 2 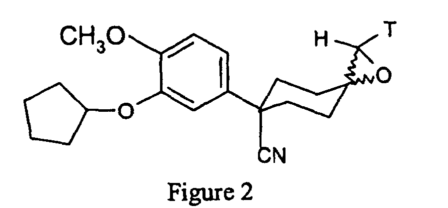 where T is CN or *p*-tolylsulfonyl.

21. A compound of formula (IV) 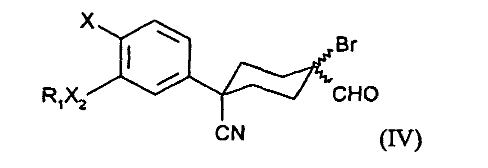 where
X is OR₂,
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O.

22. A compound of formula (IV) according to claim 21 wherein X is methoxy, R₁ is cyclopentyl and X₂ is oxygen.

23. A process for preparing a compound of formula (Ia) or (Ib) 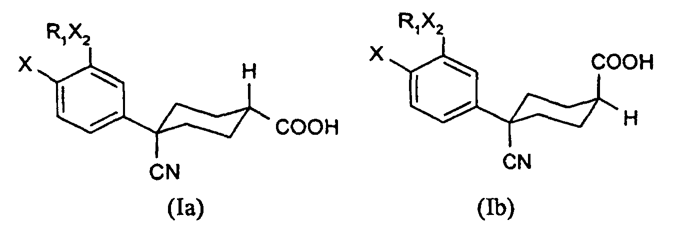 where
X is OR_{2,}
R₂ is independently selected from -CH₃ or -CH₂CH₃ optionally substituted by 1 or more halogens;
R₁ is -(CR₄R₅)ᵣR₆
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises treating an aldehyde of formula (IV) 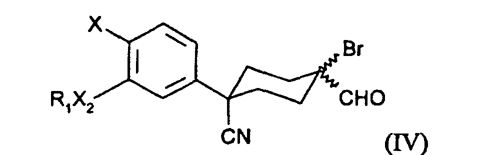 wherein X and R₁X₂ are the same as in formula I(a) and I(b) with an alkali metal cyanide.

24. The process according to claim 23 wherein the cyanide is LiCN.

25. The process according to claim 23 or 24 wherein, in formula (IV), X is methoxy, R₁ is cyclopentyl and X₂ is oxygen.

26. A process for preparing an acyl nitrile of formula (V) 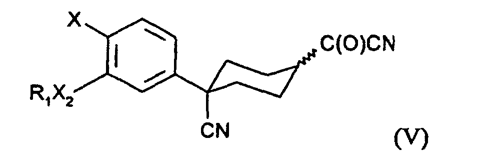 wherein
X is OR₂,
R₂ is independently selected from -CH₃ or -CH₂CH₃ optionally substituted by 1 or more halogens;
R₁ is -(CR₄R₅)ᵣR₆,
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O;
which process comprises treating an aldehyde of formula (IV) 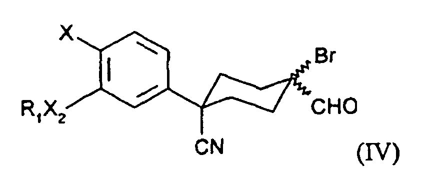 wherein X, and R₁X₂ are the same as in formula (V) with LiCN and a catalytic amount of dimethyl formamide.

27. A compound according to formula (VI) 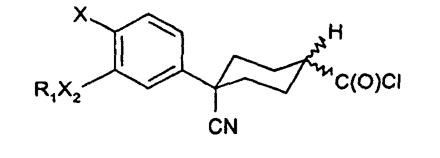 wherein
X is OR₂,
R₂ is independently selected from -CH₃ or -CH₂CH₃ optionally substituted by 1 or more halogens;
R₁ is -(CR₄R₅)ᵣR_{6,}
r is 0 to 6,
R₄ and R₅ are independently selected from hydrogen or a C₁₋₂ alkyl;
R₆ is C₃₋₆ cycloalkyl which may be optionally substituted by 1 to 3 methyl groups or one ethyl group; and
X₂ is O.

28. A compound according to claim 27 wherein X is cyclopentyloxy, and R₂ is -CH₃.

## Patentansprüche

1. Verfahren zur Herstellung einer Verbindung der Formel (I): 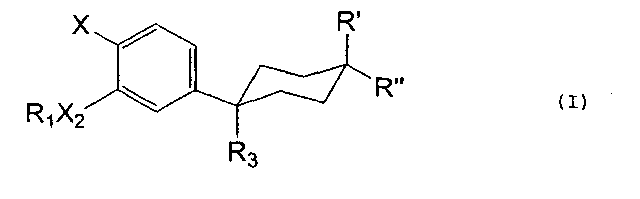 worin
R₁ -(CR₄R₅)ᵣR₆ ist, worin die Alkyleinheiten unsubstituiert oder mit einem oder mehreren Halogenen substituiert sein können;
r 0 bis 6 ist;
R₄ und R₅ unabhängig aus Wasserstoff und C₁₋₂-Alkyl ausgewählt sind;
R₆ C₃₋₆-Cycloalkyl ist, worin die Cycloalkylgruppe unsubstituiert oder mit 1 bis 3 Methylgruppen oder einer Ethylgruppe substituiert sein kann;
X YR₂ ist;
x₂ O ist;
Y O oder S(O)_{m'} ist;
m' 0, 1 oder 2 ist;
R₂ unabhängig aus -CH₃ und -CH₂CH₃ ausgewählt ist, unsubstituiert oder mit einem oder mehreren Halogenen substituiert;
R₃ CN ist; und
R' und R" unabhängig Wasserstoff oder -C(O)OH sind;
wobei das Verfahren umfaßt:
a) Umsetzen einer Verbindung der Formel (II): 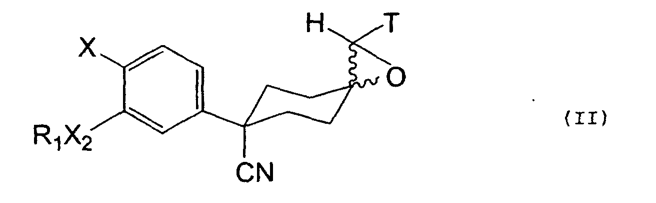 worin R₁, R₃, X₂ und X die gleichen wie in Formel (I) sind und T CN oder SO₂R ist, worin R C₁₋₆-Alkyl oder C₀₋₃-Alkylphenyl ist, mit wenigstens circa 1,5 Äquivalenten einer Lewis-Säure bei einer Temperatur zwischen circa 60 und 100°C, gegebenenfalls unter einer inerten Atmosphäre;
worin die Verbindung der Formel (II) hergestellt wird durch:
b) Umsetzen eines Ketons der Formel (A): 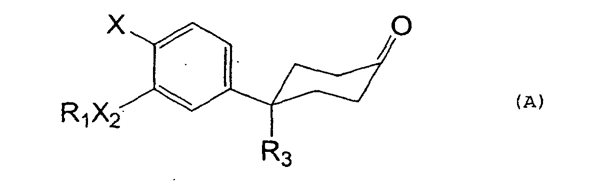 worin R₁, R₃, X₂ und X die gleichen wie in Formel (I) sind, mit einem molaren Überschuß von Chloracetonitril, einer anorganischen Base und einer katalytischen Menge von Benzyltriethylammoniumchlorid in einem wassermischbaren Lösungsmittel;
worin die Verbindung der Formel (A) hergestellt wird durch:
c) Decarboxylieren eines beta-Ketoesters der Formel (B): 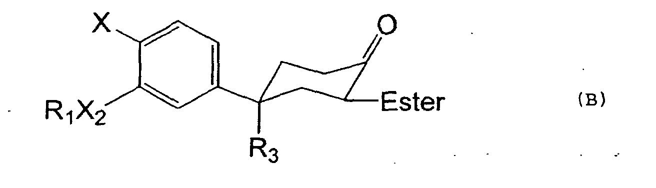 worin R₁, R₃, X₂ und X die gleichen wie in Formel (I) sind, durch Erwärmen einer Lösung des beta-Ketoesters mit Dimethylsulfoxid auf circa 150°C;
worin der beta-Ketoester der Formel (B) hergestellt wird durch:
d) Cyclisieren eines Pimelats der Formel (C): 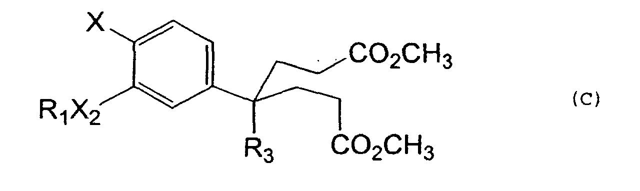 worin R₁, R₃, X₂ und X die gleichen wie in Formel (I) sind, mit circa 2 Äquivalenten einer starken Base bei circa 75°C, worin die Verbindung der Formel (C) hergestellt wird durch:
e) Umsetzen eines Nitrils der Formel (D): 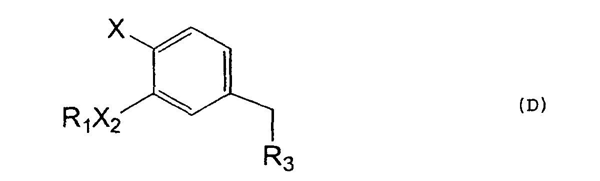 worin R₁, R₃, X₂ und X die gleichen wie in Formel (I) sind, mit einem Alkylacrylat in einem 3- bis 4-fachen molaren Überschuß in Gegenwart einer katalytischen Menge von Triton-B, worin Verbindungen der Formel (D) hergestellt werden durch:
f) Umsetzen einer Verbindung der Formel (E): 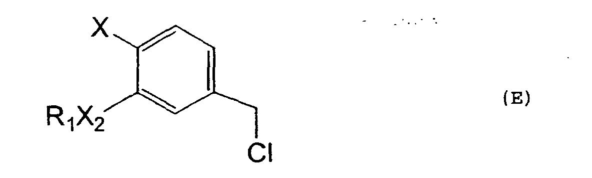 worin R₁, X₂ und X die gleichen wie in Formel (I) sind, mit einem circa 50 %igen molaren Überschuß eines Alkalimetallcyanids, worin Verbindungen der Formel (E) hergestellt werden durch:
g) Umsetzen eines Alkohols der Formel (F): 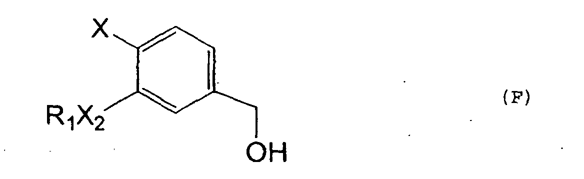 worin R₁, X₂ und X die gleichen wie in Formel (I) sind, mit überschüssiger Salzsäure, worin Verbindungen der Formel (F) hergestellt werden durch:
h) Reduzieren eines Aldehys der Formel (G): 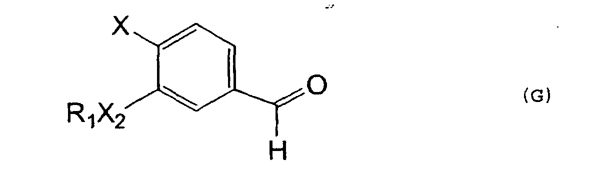
worin R₁, X₂ und X die gleichen wie in Formel (I) sind, worin die Reduktion unter Verwendung eines anorganischen Reduktionsmittels durchgeführt wird.

2. Verfahren gemäß Anspruch 1, worin in Schritt a) die Lewis-Säure Lithiumbromid ist, wenigstens circa 2 Äquivalente Wasser vorhanden sind und die Reaktion auf circa 90-95°C für bis zu circa 8 Stunden erwärmt wird.

3. Verfahren gemäß Anspruch 1, worin in Schritt b) die anorganische Base wäßriges Kaliumhydroxid ist und das wassermischbare Lösungsmittel Tetrahydrofuran ist und die Reaktion bei einer Temperatur von circa 0°C durchgeführt wird.

4. Verfahren gemäß Anspruch 1, worin in Schritt c) die starke Base Natriummethoxid ist, das mit circa 1,5 molaren Äquivalenten vorhanden ist, und die Reaktion bei circa 70 bis 75°C für bis zu 2 Stunden durchgeführt wird.

5. Verfahren gemäß Anspruch 1, worin Schritte c) und d) durch Cyclisieren des Pimelats kombiniert werden, indem es mit Natriummethoxid umgesetzt wird, der resultierende beta-Ketoester ohne Isolieren decarboxyliert wird, indem Dimethylsulfoxid zur esterhaltigen Lösung gegeben wird, und die Lösung auf circa 145°C für bis zu 2 Stunden erwärmt wird.

6. Verfahren gemäß Anspruch 5, worin die Reaktionsmischung nach Beendigung des Cyclisierungsreaktionsschrittes auf circa Raumtemperatur abgekühlt und der pH auf 6,8-7,2 eingestellt wird, bevor Dimethylsulfoxid zugegeben wird.

7. Verfahren gemäß Anspruch 1, Schritt e), worin das Alkylacrylat Methylacrylat ist.

8. Verfahren gemäß Anspruch 1, Schritt f), worin das Alkalimetallcyanid Natriumcyanid ist und die Reaktion bei circa 55°C durchgeführt wird.

9. Verfahren gemäß Anspruch 1, Schritt h), worin das Reduktionsmittel Natriumborhydrid ist.

10. Verfahren gemäß einem der Ansprüche 1 bis 9, worin in Formel (I) R₁ Cyclopentyl ist; Y Sauerstoff ist; und R₂ Methyl ist.

11. Verfahren zur Herstellung einer Verbindung der Formel (Ia) oder (Ib) oder einer Mischung daraus: 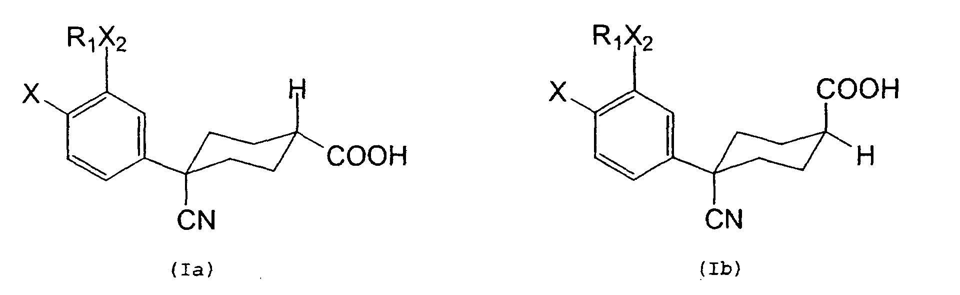 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist;
wobei das Verfahren das Behandeln eines Acylnitrils der Formel (V) mit Wasser umfaßt: 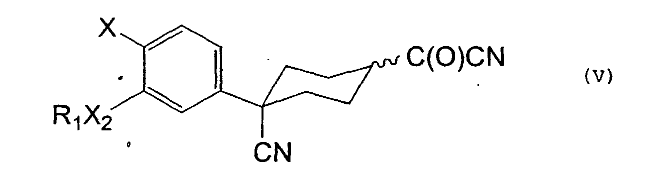 worin X und R₁X₂ die gleichen wie in Formel (Ia) und (Ib) sind.

12. Verfahren gemäß Anspruch 11, worin die Verbindung der Formel (V) diejenige der Formel (Va) ist: 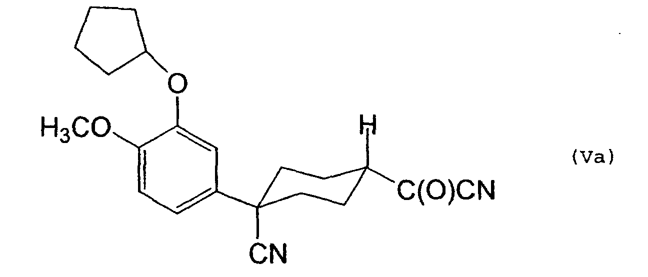 und zur Bildung einer Verbindung der Formel (Ia) führt.

13. Verfahren gemäß Anspruch 11, worin die Verbindung der Formel (V) diejenige der Formel (Vb) ist: 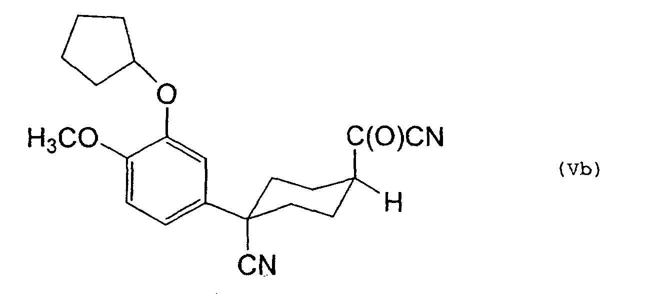 und zur Bildung einer Verbindung der Formel (Ib) führt.

14. Verfahren zur Herstellung einer Verbindung der Formel (V): 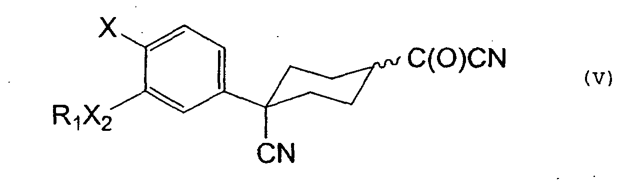 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist;
wobei das Verfahren das Halten einer Verbindung der Formel (Z) bei etwa Raumtemperatur in Lösung für eine ausreichende Zeit umfaßt, um die Umwandlung zu bewirken: 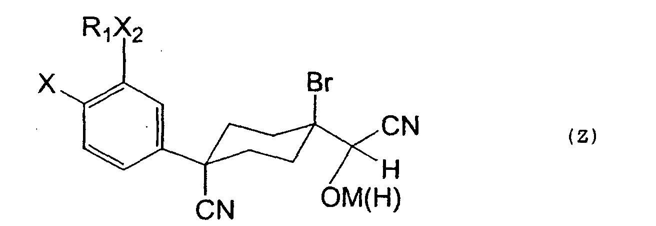 worin X und R₁X₂ die gleichen wie in Formel (V) sind und M Li oder Mg ist und die Gruppe (H) Wasserstoff ist.

15. Verfahren gemäß Anspruch 14, worin die Formel (Z) diejenige der Figur 1 ist: 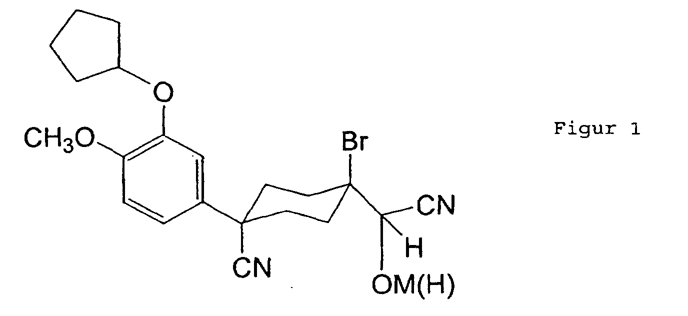 worin M Li oder Mg ist und (H) Wasserstoff ist.

16. Verbindung der Formel (Z): 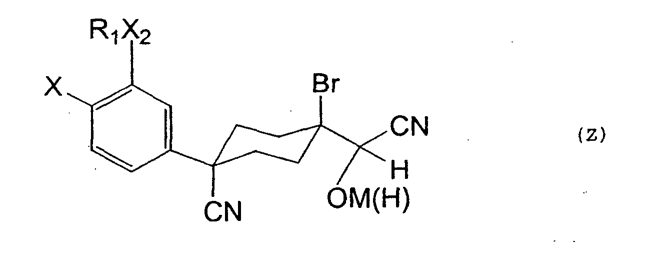 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist.

17. Verbindung der Formel (Z) gemäß Anspruch 16, die: 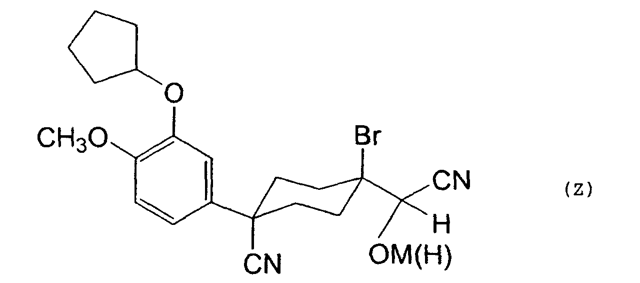 worin M Li oder Mg ist und (H) Wasserstoff ist.

18. Verfahren zur Herstellung einer Verbindung der Formel (Z): 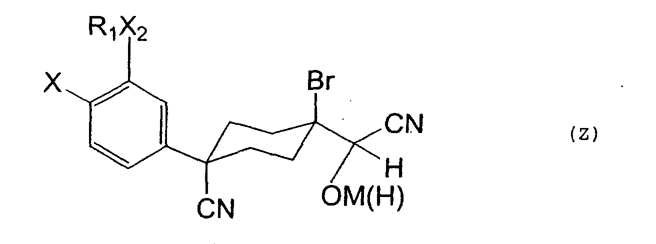 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist;
wobei das Verfahren das Behandeln eines Epoxids der Formel (II): 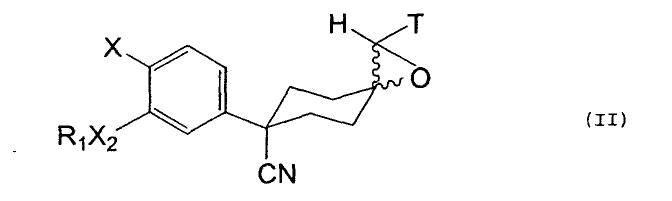 worin X und R₁X₂ die gleichen wie in Formel (Z) sind und T CN oder SO₂R ist, worin R C₁₋₆-Alkyl oder C₀₋₃-Alkylphenyl ist, mit einer Lewis-Säure unter wasserfreien Bedingungen umfaßt.

19. Verfahren gemäß Anspruch 18, worin die Lewis-Säure LiBr ist.

20. Verfahren gemäß Anspruch 18 oder 19, worin das Epoxid der Formel (II) die durch Figur 2 dargestellte Struktur hat: 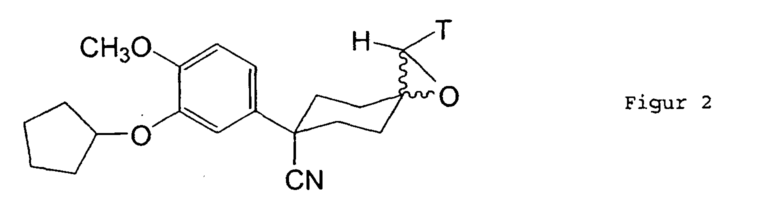 worin T CN oder p-Tolylsulfonyl ist.

21. Verbindung der Formel (IV): 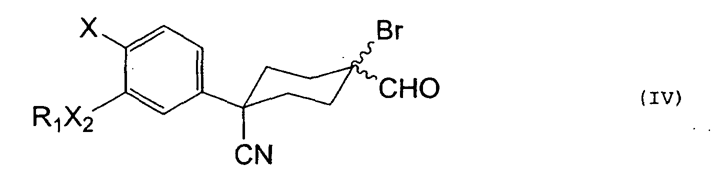 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist.

22. Verbindung der Formel (IV) gemäß Anspruch 21, worin X Methoxy ist, R₁ Cyclopentyl ist und X₂ Sauerstoff ist.

23. Verfahren zur Herstellung einer Verbindung der Formel (Ia) oder (Ib) : 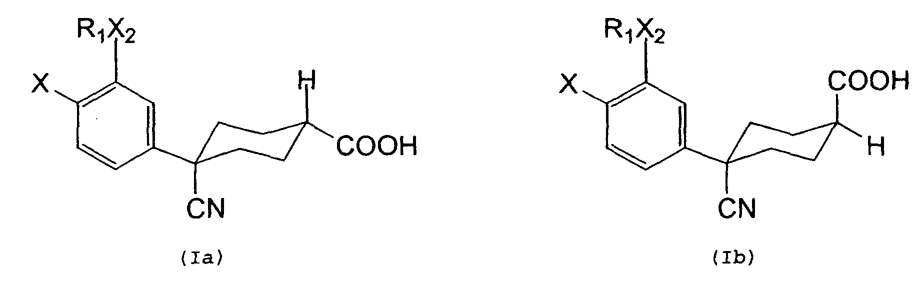 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist;
wobei das Verfahren das Behandeln eines Aldehyds der Formel (IV): 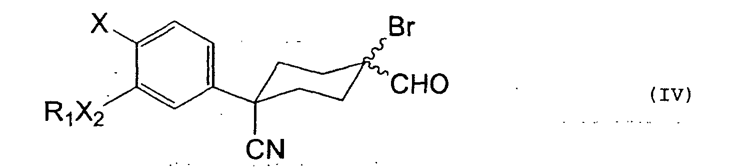 worin X und R₁X₂ die gleichen wie in Formel (Ia) und (Ib) sind, mit einem Alkalimetallcyanid umfaßt.

24. Verfahren gemäß Anspruch 23, worin das Cyanid LiCN ist.

25. Verfahren gemäß Anspruch 23 oder 24, worin in Formel (IV) X Methoxy ist, R₁ Cyclopentyl ist und X₂ Sauerstoff ist.

26. Verfahren zur Herstellung eines Acylnitrils der Formel (V): 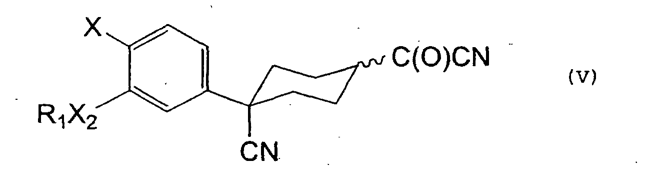 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist;
wobei das Verfahren das Behandeln eines Aldehyds der Formel (IV): 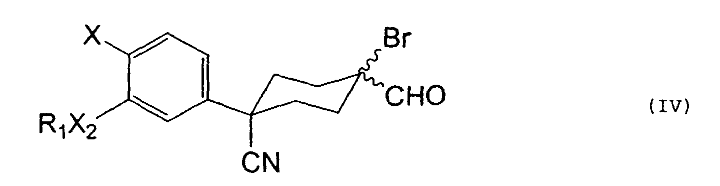 worin X und R₁X₂ die gleichen wie in Formel (V) sind, mit LiCN und einer katalytischen Menge von Dimethylformamid.

27. Verbindung gemäß Formel (VI): 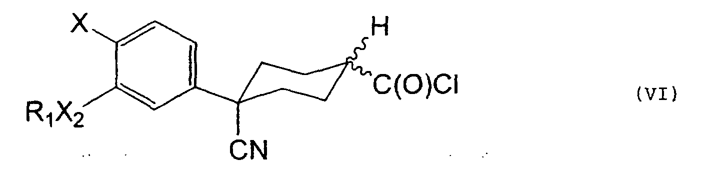 worin
X OR₂ ist;
R₂ unabhängig aus -CH₃ und -CH₂CH₃ ausgewählt ist, gegebenenfalls substituiert mit einem oder mehreren Halogenen;
R₁ -(CR₄R₅)ᵣR₆ ist;
r 0 bis 6 ist;
R₄ und R₅ unabhängig aus Wasserstoff und C₁₋₂-Alkyl ausgewählt sind;
R₆ C₃₋₆-Cycloalkyl ist, das gegebenenfalls mit 1 bis 3 Methylgruppen oder einer Ethylgruppe substituiert sein kann; und
X₂ O ist.

28. Verbindung gemäß Anspruch 27, worin X Cyclopentyloxy ist und R₂ -CH₃ ist.

## Revendications

1. Procédé de préparation d'un composé de formule (I) 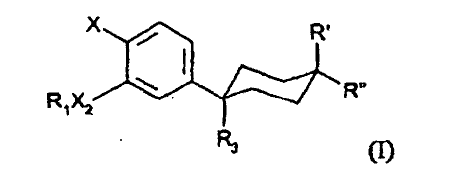 où
- R₁ représente un groupe -(CR₄R₅)ᵣR₆ dans lequel les entités alkyle peuvent être non substituées ou substituées par un ou plusieurs halogènes ;
- r est 0 à 6 ;
- R₄ et R₅ sont indépendamment choisis parmi l'hydrogène ou un groupe alkyle C₁₋₂ :
- R₆ représente un groupe cycloalkyle C₃₋₆ dans lequel le groupe cycloalkyle peut être non substitué ou substitué par 1 à 3 groupes méthyle ou un groupe éthyle ;
- X représente YR₂ ;
- X₂ représente O ;
- Y représente O ou S(O)_{m'} ;
- m' est égal à 0, 1 ou 2 ;
- R₂ est indépendamment choisi parmi -CH₃, ou -CH₂CH₃ non substitué ou substitué par 1 ou plusieurs halogènes ;
- R₃ représente CN ; et
- R' et R" représente indépendamment l'hydrogène ou -C(O)OH ; lequel procédé comprend :
(a) la réaction d'un composé de formule (II) 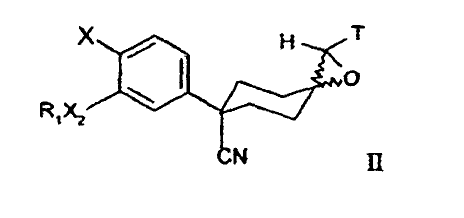 dans laquelle R₁, R₃, X₂ et X sont les mêmes que dans la formule (I), et T représente CN ou SO₂R où R est un groupe alkyle C₁₋₆, ou un groupe alkyl(C₀₋₃)phényle, avec au moins 1,5 équivalent environ d'un acide de Lewis, à une température comprise entre environ 60°C et 100°C, éventuellement sous atmosphère inerte,
dans lequel le composé de formule (II) est préparé par
(b) la réaction d'une cétone de formule (A) : 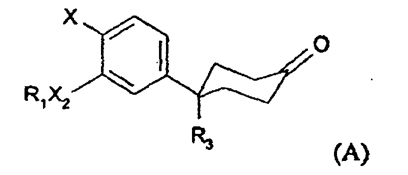 dans laquelle R₁, R₃, X₂ et X sont les mêmes que dans la formule (I), avec un excès molaire de chloroacétonitrile, une base inorganique et une quantité catalytique de chlorure de benzyltriéthylammonium dans un solvant miscible à l'eau ;
dans lequel le composé de formule (A) est préparé par
(c) la décarboxylation d'un bêta-céto-ester de formule (B) : 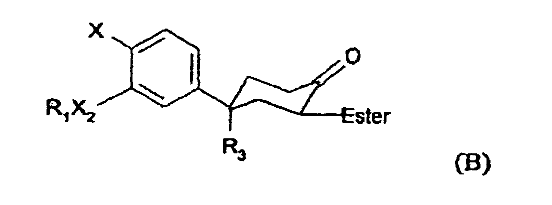 dans laquelle R₁, R₃, X₂ et X sont les mêmes que dans la formule (I), par chauffage d'une solution du bêta-céto-ester avec le diméthylsulfoxyde à environ 150°C;
dans lequel le bêta-céto-ester de formule (B) est préparé par
(d) la cyclisation d'un pimélate de formule (C) : 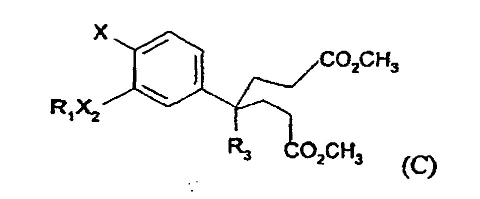 dans laquelle R₁, R₃, X₂ et X sont les mêmes que dans la formule (I), avec environ 2 équivalents d'une base forte à environ 75°C,
dans lequel le composé de formule (C) est préparé par
(e) la réaction d'un nitrile de formule (D) 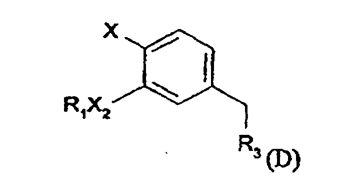 dans laquelle R₁, R₃, X₂ et X sont les mêmes que dans la formule (I), avec un acrylate d'alkyle en excès molaire de 3 à 4 fois, en présence d'une quantité catalytique de Triton-B,
dans lequel les composés de formule (D) sont préparés par
(f) la réaction d'un composé de formule (E) 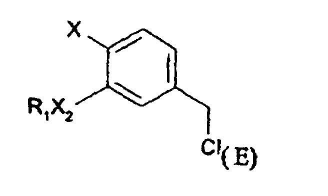 dans laquelle R₁, X₂ et X sont les mêmes que dans la formule (I), avec un cyanure de métal alcalin en excès molaire d'environ 50 %,
dans lequel les composés de formule (E) sont préparés par
(g) la réaction d'un alcool de formule (F) 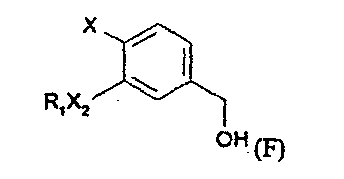 dans laquelle R₁, X₂ et X sont les mêmes que dans la formule (I), avec un excès d'acide chlorhydrique,
dans lequel les composés de formule (F) sont préparés par
(h) la réduction d'un aldéhyde de formule (G) 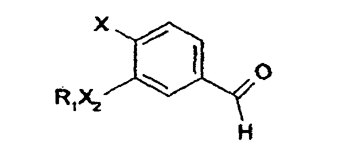
dans laquelle R₁, X₂ et X sont les mêmes que dans la formule (I), où la réduction est effectuée en utilisant un agent réducteur inorganique.

2. Procédé selon la revendication 1, dans lequel, à l'Etape (a), l'acide de Lewis est le bromure de lithium, au moins 2 équivalents environ d'eau sont présents, et le mélange réactionnel est chauffé à environ 90-95°C pendant jusqu'à environ 8 heures.

3. Procédé selon la revendication 1, dans lequel, à l'Etape (b), la base inorganique est l'hydroxyde de potassium aqueux et le solvant miscible à l'eau est le tétrahydrofurane, et la réaction est effectuée à une température d'environ 0°C.

4. Procédé selon la revendication 1, dans lequel, à l'Etape (c), la base forte est le méthoxyde de sodium qui est présent à raison d'environ 1,5 équivalent molaire, et la réaction est effectuée à environ 70 à 75°C pendant jusqu'à 2 heures.

5. Procédé selon la revendication 1, dans lequel les Etapes (c) et (d) sont combinées par la cyclisation du pimélate en faisant réagir celui-ci avec le méthoxyde de sodium, en décarboxylant le bêta-céto-ester obtenu sans l'isoler, en ajoutant du diméthylsulfoxyde à la solution contenant l'ester et en chauffant cette solution à environ 145°C pendant jusqu'à 2 heures.

6. Procédé selon la revendication 5, dans lequel, une fois que l'étape de réaction de cyclisation est terminée, le mélange réactionnel est refroidi approximativement à la température ambiante et le pH est ajusté à 6,8-7,2 avant l'addition du diméthylsulfoxyde.

7. Procédé selon la revendication 1, étape (e), dans laquelle l'acrylate d'alkyle est l'acrylate de méthyle.

8. Procédé selon la revendication 1, étape (f), dans laquelle le cyanure de m étal alcalin est le cyanure de sodium et la réaction est effectuée à environ 55°C.

9. Procédé selon la revendication 1, étape (h), dans laquelle l'agent réducteur est le borohydrure de sodium.

10. Procédé selon l'une quelconque des revendications 1 à 9, dans lequel, dans la formule (I), R₁ représente le groupe cyclopentyle ; Y représente l'oxygène ; et R₂ représente le groupe méthyle.

11. Procédé de préparation d'un composé de formule (Ia) ou d'un composé de formule (Ib) ou d'un mélange de ceux-ci 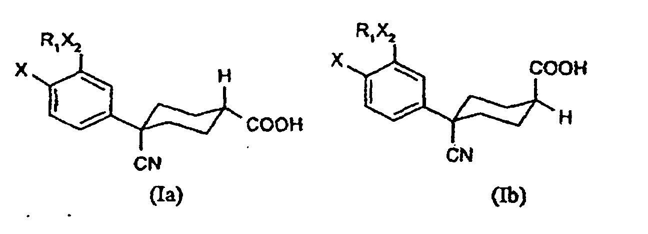 dans lesquelles
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O ;
lequel procédé comprend le traitement d'un acylonitrile de formule (V) avec l'eau 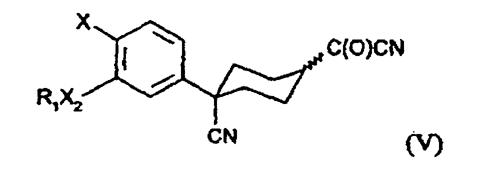 où X et R₁X₂ sont les mêmes que dans les formules (Ia) et (Ib).

12. Procédé selon la revendication 11, dans lequel le composé de formule (V) est celui de formule (Va) 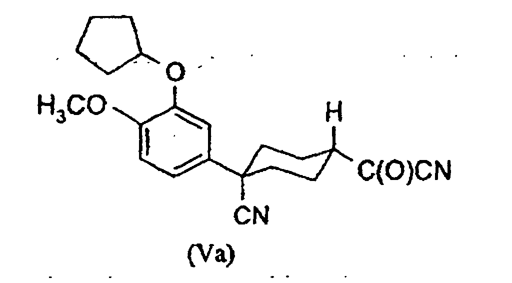 et entraîne la formation d'un composé de formule (Ia)

13. Procédé selon la revendication 11, dans lequel le composé de formule (V) est celui de formule (Vb) 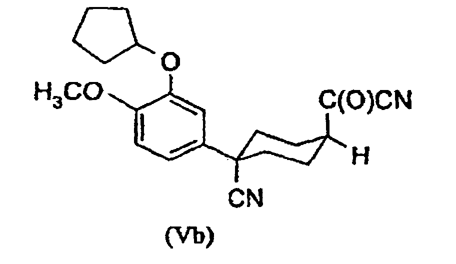 et entraîne la formation d'un composé de formule (Ib).

14. Procédé de préparation d'un composé de formule (V) 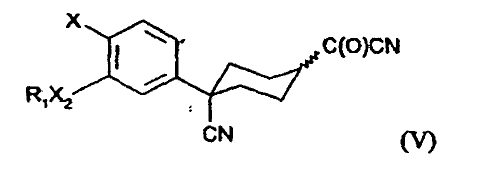 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O ;
lequel procédé comprend le maintien en solution à approximativement la température ambiante, pendant un temps suffisant pour effectuer la conversion, d'un composé de formule (Z) 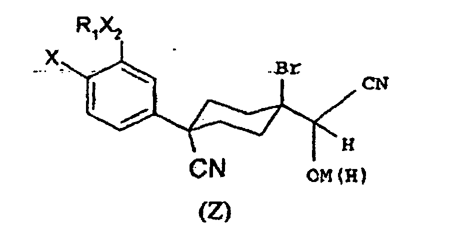 dans laquelle X et R₁X₂ sont les mêmes que dans la formule (V) et M représente Li ou Mg et le groupe (H) représente l'hydrogène.

15. Procédé selon la revendication 14, dans lequel la formule (Z) est celle représentée en Figure 1 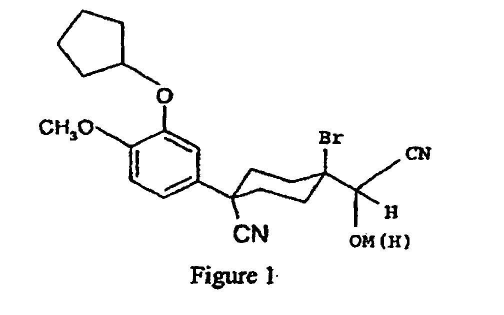 où M représente Li ou Mg et (H) représente l'hydrogène.

16. Composé de formule (Z) 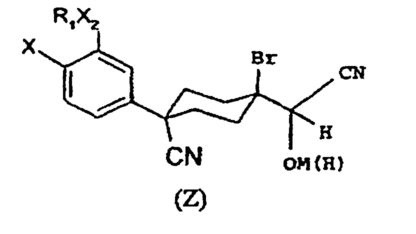 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est de 0 à 6,
- R₄ et R₅ sont choisis indépendamment entre l'hydrogène ou un groupe alkyle C₁₋₂ ;
- R₆ représente un groupe cycloalkyle C₃₋₆ qui peut être éventuellement substitué par 1 à 3 groupes méthyle ou un groupe éthyle ; et
- X₂ représente O.

17. Composé de formule (Z) selon la revendication 16, qui est 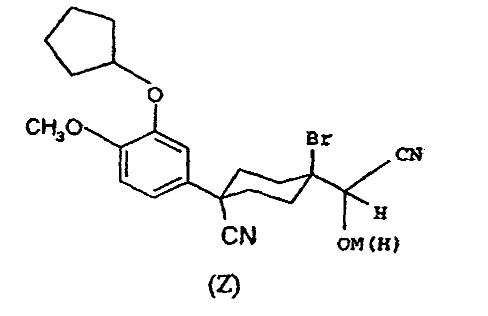 dans laquelle M représente Li ou Mg et (H) représente l'hydrogène.

18. Procédé de préparation d'un composé de formule (Z) 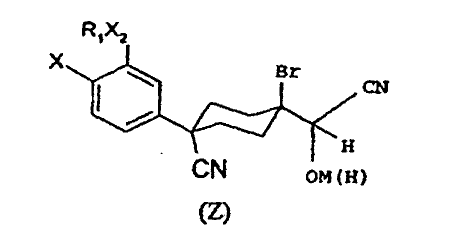 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est égal de 0 à 6,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O.
lequel procédé comprend le traitement d'un époxyde de formule (II) 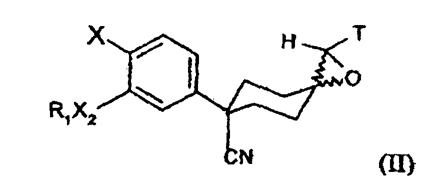 dans laquelle X et R₁X₂ sont les mêmes que dans la formule (Z), et T représente CN ou SO₂R où R représente un groupe alkyle C₁₋₆ ou un groupe alkyl(C₀₋₃)phényle, avec un acide de Lewis dans des conditions anhydres.

19. Procédé selon la revendication 18, dans lequel l'acide de Lewis est LiBr.

20. Procédé selon la revendication 18 ou la revendication 19, dans lequel un époxyde de formule (II) a la structure représentée en Figure 2 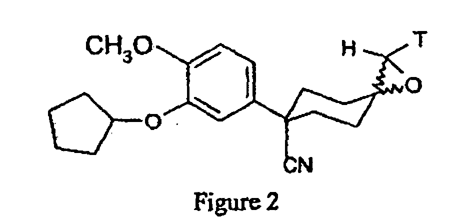 où T représente un groupe CN ou *p*-tolylsulfonyle.

21. Composé de formule (IV) 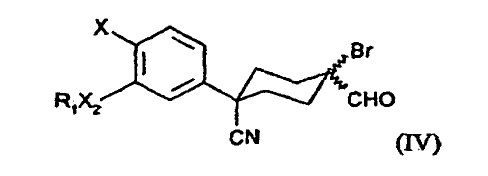 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est de 0 à 6,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O.

22. Composé de formule (IV) selon la revendication 21, dans laquelle X représente un groupe méthoxy, R₁ représente un groupe cyclopentyle et X₂ représente l'oxygène.

23. Procédé de préparation d'un composé de formule (Ia) ou de formule (Ib) 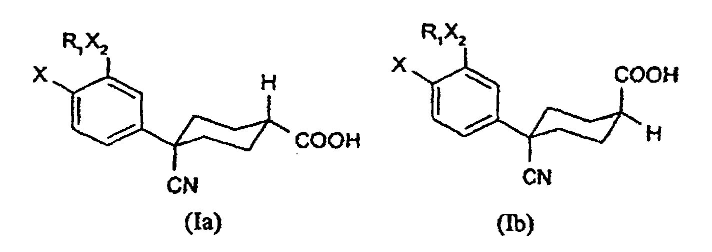 où
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est de 0 à 6,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O,
lequel procédé comprend le traitement d'un aldéhyde de formule (IV) 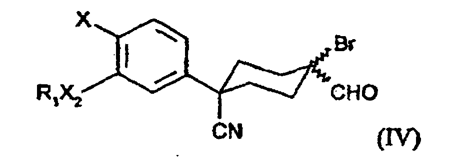 dans laquelle X et R₁X₂ sont les mêmes que dans la formule (Ia) et (Ib), avec un cyanure de métal alcalin.

24. Procédé selon la revendication 23, dans lequel le cyanure est LiCN.

25. Procédé selon la revendication 23 ou la revendication 24, dans lequel, dans la formule (IV), X représente un groupe méthoxy, R₁ représente un groupe cyclopentyle et X₂ représente l'oxygène.

26. Procédé de préparation d'un acylonitrile de formule (V) 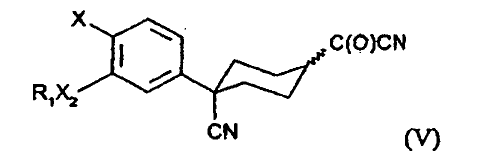 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est de 0 à 6,
- R₄ et R₅ sont choisis indépendamment entre l'hydrogène ou un groupe alkyle C₁₋₂;
- R₆ représente un groupe cycloalkyle C₃₋₆ qui peut être éventuellement substitué par 1 à 3 groupes méthyle ou un groupe éthyle ; et
- X₂ représente O,
lequel procédé comprend le traitement d'un aldéhyde de formule (IV) 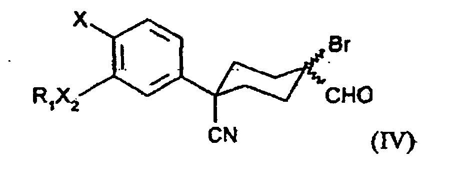 dans laquelle X et R₁X₂ sont les mêmes que dans la formule (V), avec LiCN et une quantité catalytique de diméthylformamide.

27. Composé selon la formule (VI) 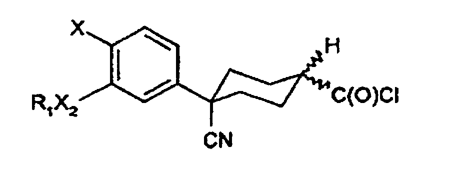 dans laquelle
- X représente OR₂,
- R₂ est choisi indépendamment entre -CH₃ ou -CH₂CH₃ éventuellement substitué par 1 ou plusieurs halogènes ;
- R₁ représente -(CR₄R₅)ᵣR₆,
- r est de 0 à 6,
- R₄ et R₅ sont choisis indépendamment entre l'hydrogène ou un groupe alkyle C₁₋₂ ;
- R₆ représente un groupe cycloalkyle C₃₋₆ qui peut être éventuellement substitué par 1 à 3 groupes méthyle ou un groupe éthyle ; et
- X₂ représente O.

28. Composé selon la revendication 27, dans lequel X représente un groupe cyclopentyloxy et R₂ représente le groupe -CH₃.
